# FASCICULE DE BREVET EUROPEEN

(11) **EP 0 863 896 B1**
(45) Date de publication et mention de la délivrance du brevet: **09.02.2005**
(21) Numéro de dépôt: 96938267.0
(22) Date de dépôt: 07.11.1996
(51) Int. Cl.: C07D 405/06, A61K 31/415, A61K 31/36

(54) **NOUVEAUX DERIVES DE L'IMIDAZOLE, PROCEDE DE PREPARATION, COMPOSITIONS PHARMACEUTIQUES ET LEUR UTILISATION COMME ANTAGONISTES DE L'ENDOTHELINE**
NEUE IMIDAZOLDERIVATE, VERFAHREN ZUR HERSTELLUNG, PHARMAZEUTISCHE ZUSAMMENSETZUNGEN UND IHRE VERWENDUNG ALS ENDOTHELIN ANTAGONISTEN
NOVEL IMIDAZOLE DERIVATIVES, METHOD FOR PREPARING SAME, PHARMACEUTICAL COMPOSITIONS AND USE OF SAID DERIVATIVES AS ENDOTHELIN ANTAGONISTS

(30) Priorité: 08.11.1995 FR 9513190
(43) Date de publication de la demande: 16.09.1998
(73) Titulaire: Aventis Pharma S.A., 92160 Antony (FR)
(72) Inventeur: HECKMANN, Bertrand, F-94230 Cachan (FR); JOUQUEY, Simone, F-75020 Paris (FR); VEVERT, Jean-Paul, F-93500 Pantin (FR); ZHANG, Jidong, F-75013 Paris (FR)
(86) Numéro de dépôt international: PCT/FR1996/001750
(87) Numéro de publication internationale: WO 1997/017340

(56) Documents cités:
- WO-A-94/14434
- WO-A-96/04276

## Description

La présente invention concerne de nouveaux dérivés de l'imidazole, leur procédé de préparation, les nouveaux intermédiaires obtenus, leur application à titre de médicaments, les compositions pharmaceutiques les renfermant et la nouvelle utilisation de dérivés de l'imidazole.

La présente invention a pour objet les produits de formule (I) : 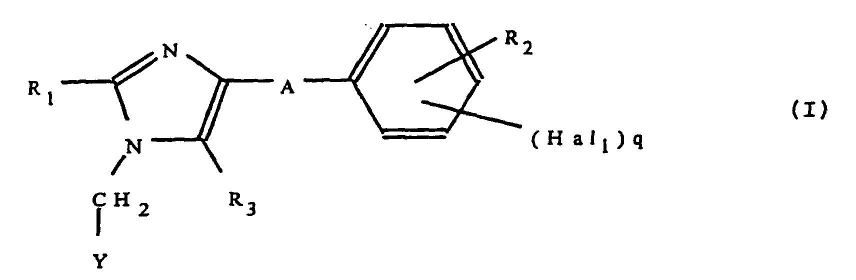 dans laquelle :
R₁ représente un radical éthyle, n-propyle, isopropyle, n-butyle, isobutyle ou terbutyle,
A représente un atome de soufre,
R₂ représente un radical alkyle ou alcoxy, linéaires ou ramifiés, renfermant au plus 4 atomes de carbone et substitués par un radical carboxy libre, salifié ou estérifié,
q est égal à 0,
R₃, représente un radical carboxy libre, salifié ou estérifié, ou un radical tétrazolyle libre ou salifié,
Y représente un radical phényle substitué par un radical dioxol et éventuellement par un atome d'halogène,
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

Dans les produits de formule (I) et dans ce qui suit :
- le terme radical alkyle linéaire ou ramifié désigne les radicaux méthyle, éthyle, propyle, isopropyle, butyle, isobutyle, sec-butyle, tert-butyle, pentyle, isopentyle, hexyle, isohexyle et également heptyle, octyle, nonyle et décyle ainsi que leurs isomères de position linéaires ou ramifiés,
- le terme radical alcoxy linéaire ou ramifié désigne les radicaux méthoxy, éthoxy, propoxy, isopropoxy, butoxy linéaire, secondaire ou tertiaire, pentoxy ou hexoxy ainsi que leurs isomères de position linéaires ou ramifiés,
- le terme atome d'halogène désigne de préférence l'atome de chlore, mais peut aussi représenter un atome de fluor, de brome ou d'iode, Hall représente de préférence un atome de fluor.

Le ou les radicaux carboxy des produits de formule (I) peuvent être salifiés ou estérifiés par les groupements divers connus de l'homme du métier parmi lesquels on peut citer, par exemple :
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxycarbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle ou phénéthyle.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoique, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

La présente invention a particulièrement pour objet les produits suivants :
- acide 4-((4-(carboxyméthoxy) phényl) thio)-1-((6-chloro-1,3-benzodioxol-5-yl) méthyl)-2-propyl-1H-imidazole-5-carboxylique,
- acide 4-((4-(2-carboxyéthyl) phényl) thio)-1-((6-chloro-1,3-benzodioxol-5-yl) méthyl)-2-(1-méthyléthyl)-1H-imidazole-5-carboxylique,
- acide 4-((4-carboxyphényl) thio) 1-((6-chloro 1,3-benzodioxol-5-yl) méthyl) 2-propyl 1H-imidazole 5-carboxylique.

Dans les produits de formule (I) et dans ce qui suit :
- le terme radical alkylthio désigne les radicaux dans lesquels le radical alkyle est tel que défini ci-dessus comme par exemple dans méthylthio, éthylthio, propylthio, isopropylthio, butylthio, sec-butylthio, tert-butylthio, isopentylthio, isohexylthio, mais aussi heptylthio, octylthio, nonylthio ou décylthio ainsi que leurs isomères de position linéaires ou ramifiés.

La présente invention a aussi pour objet le procédé de préparation des produits de formule (I), telle-que définie ci-dessus, caractérisé en ce que :
soit l'on soumet un composé de formule (II) : 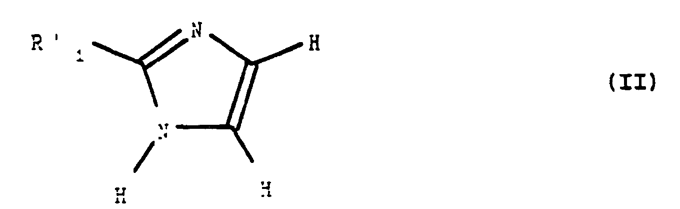 dans laquelle R'₁ a la signification indiquée ci-dessus pour R₁, dans laquelle les éventuelles fonctions réactives sont éventuellement protégées par des groupements protecteurs, à une réaction avec un composé de formule (III) : 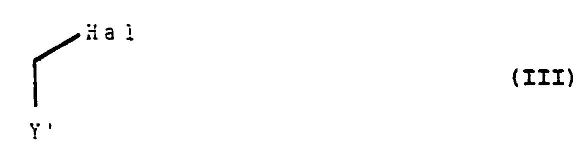 dans laquelle Hal représente un atome d'halogène, et
   Y' a la signification indiquée ci-dessus pour Y, dans laquelle les éventuelles fonctions réactives sont éventuellement protégées par des groupements protecteurs,-
   pour obtenir le produit de formule (IV) : 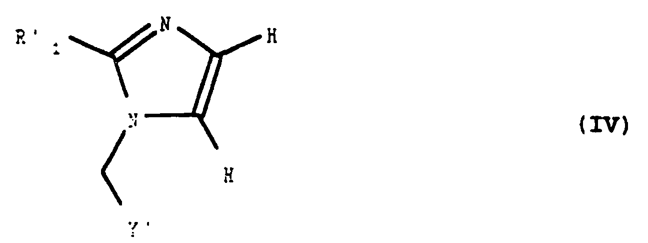 dans laquelle R'₁ et Y' ont les significations indiquées ci-dessus, produit de formule (IV) que l'on peut soumettre à une réaction d'halogénation, pour obtenir le produit de formule (V) : 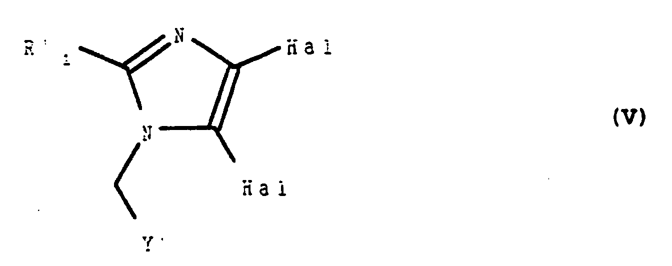 dans laquelle R'₁, Hal et Y' ont les significations indiquées ci-dessus,
soit l'on soumet un composé de formule (VI) : 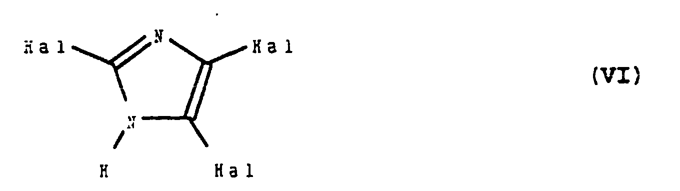 dans laquelle Hal a la signification indiquée ci-dessus,
   soit à une réaction avec le composé de formule (III) telle que définie ci-dessus, soit à l'action d'un groupement protecteur P, pour obtenir un produit de formule (VII) : 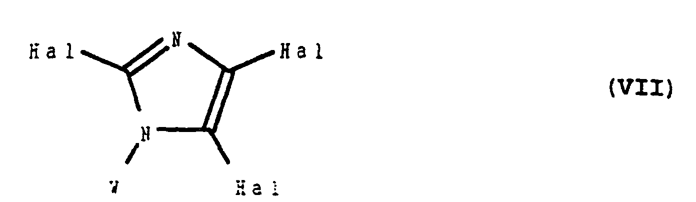 dans laquelle Hal a la signification indiquée ci-dessus et W représente soit -CH₂-Y' avec Y' tel que défini ci-dessus, soit P qui représente un groupement protecteur de l'atome d'azote, produit de formule (VII) que l'on soumet à une réaction d'échange halogène-métal puis à une réaction avec le diméthylformamide ou avec un électrophile de formule (VIIIₐ) ou (VIII_{b}) :

   L₁-CHO (VIIIₐ)

   L₁-CO-Cl (VIII_{b})

   dans lesquelles L₁ représente un radical alkyle, linéaire ou ramifié, renfermant au plus 6 atomes de carbone et éventuellement substitué par un radical alcoxy ou hydroxyle protégé, pour obtenir un produit de formule (IX) : 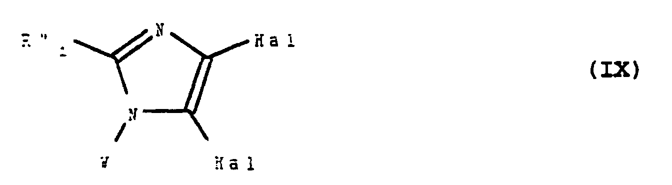 dans laquelle Hal et W ont les significations indiquées ci-dessus, R"₁ représente un radical alkyl-carbonyle, formyle ou hydroxyalkyle dans lesquels le radical alkyle a la signification indiquée ci-dessus et dans lesquels les éventuelles fonctions réactives sont éventuellement protégées par des groupements protecteurs, produits de formules (V) et (IX) que l'on peut soumettre à une réaction d'échange halogène-métal sur l'un des atomes d'halogène puis à une réaction avec CO₂ ou diméthylformamide ou un composé électrophile de formule (X) : 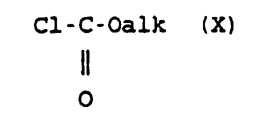 dans laquelle alk représente un radical alkyle renfermant au plus 4 atomes de carbone,
   pour obtenir le composé de formule (XI) : 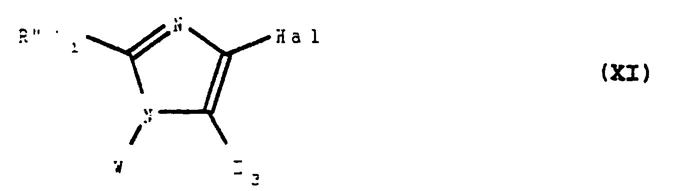 dans laquelle R'' '₁ représente R'₁ ou R"₁ tels que définis ci-dessus, Hal, Y' et W ont les significations indiquées ci-dessus et Z₃ représente le radical carboxy, libre ou estérifié ou le radical formyle,
   composé de formule (XI) que l'on peut soumettre à une réaction avec un composé de formule (XII) : 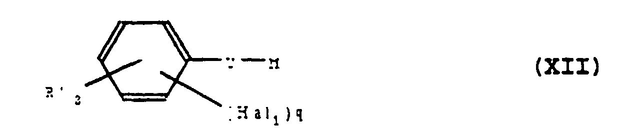 dans laquelle U représente un atome de soufre ou d'oxygène, M représente un métal tel que sodium, potassium ou cuivre, Hal₁ et q ont la signification indiquée ci-dessus, R'₂ représente R₂ tel que défini ci-dessus, dans lequel les éventuelles fonctions réactives sont éventuellement protégées par des groupements protecteurs pour obtenir le composé de formule (XIII) : 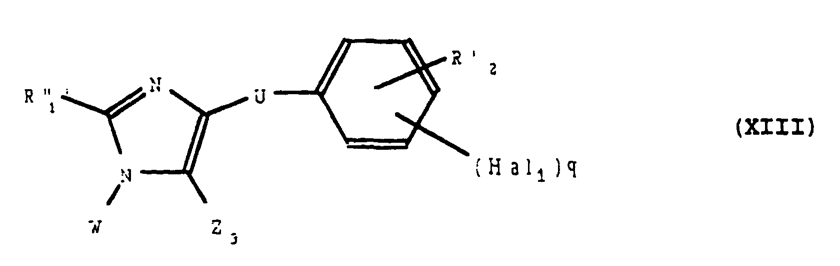 dans laquelle R'' '₁, U, R'₂ Hal₁, q et W ont les-significations indiquées ci-dessus, produit de formule (XIII) que lorsque Z₃ représente le radical formyle, l'on peut soumettre à une réaction d'oxydation pour obtenir le produit de formule (XIV) : 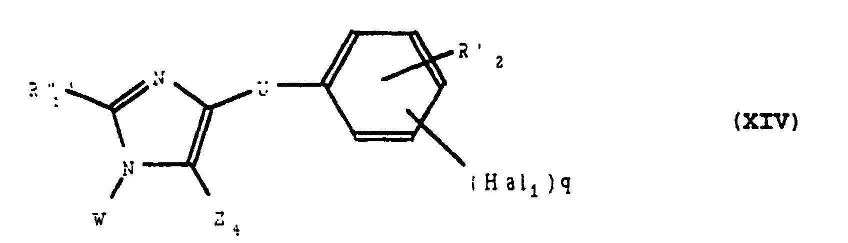 dans lequel R'' '₁, U, R'₂, Hal₁, q et W ont les significations indiquées ci-dessus, et Z₄ représente le radical cyano ou le radical carboxy libre ou estérifié par un radical alkyle linéaire ou ramifié renfermant au plus 6 atomes de carbone, produits de formule (XIII) ou (XIV) que, dans le cas où W représente P tel que défini ci-dessus et après libération de la fonction amine bloquée par P tel que défini ci-dessus, l'on fait réagir avec le composé de formule (III) telle que définie ci-dessus, pour obtenir un produit de formule (XV) : 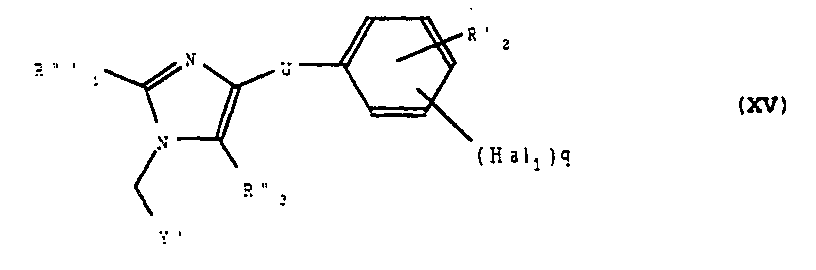 dans laquelle R'' '₁, U, R'₂, Hal₁, q et Y' ont les significations indiquées ci-dessus, et R"₃ représente Z₃ ou Z₄ tels que définis ci-dessus,
soit l'on soumet un composé de formule (XX) :

   Y'-CHO (XX)
dans laquelle Y' a la signification indiquée ci-dessus, à un composé de formule (XXI) : 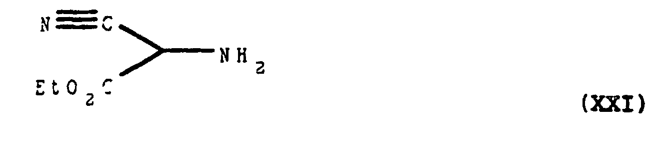 pour obtenir un produit de formule (XXII) : 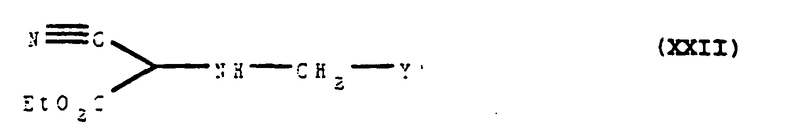 dans laquelle Y' a la signification indiquée ci-dessus, que l'on soumet à un composé de formule (XXIII) : 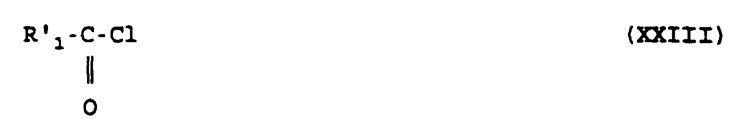 dans laquelle R'₁ a la signification indiquée ci-dessus, pour obtenir le produit de formule (XXIV) : 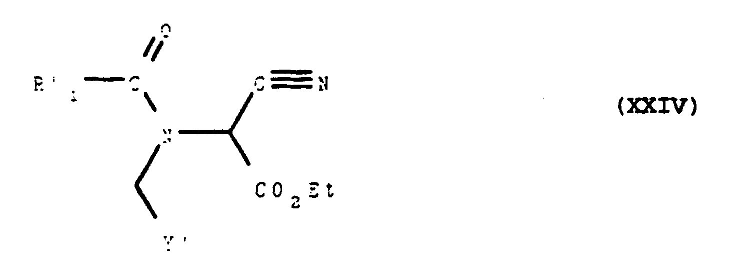 dans laquelle Y' et R'₁ ont les significations indiquées ci-dessus, que l'on soumet à un composé de formule (XXV) : 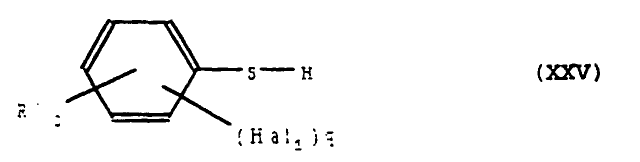 dans laquelle R'₂, Hal₁ et q ont la signification indiquée ci-dessus pour obtenir le produit de formule (XXVI) : 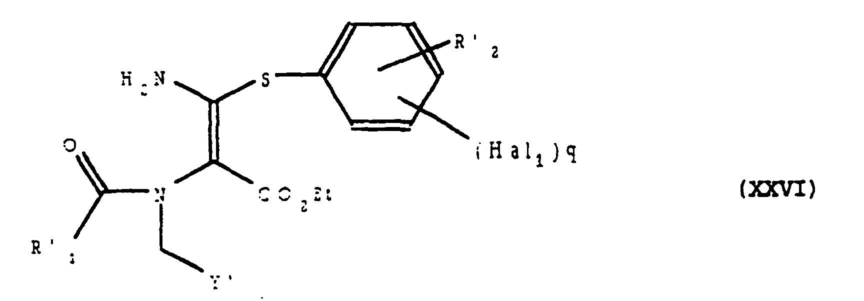 dans laquelle Y', R'₁, R'₂, Hal₁ et q ont les significations indiquées ci-dessus,
qui est cyclisé en produit de formule (I₂) : 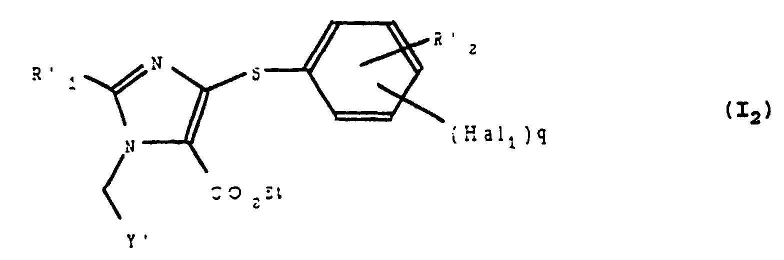 dans laquelle Y', R'₁, R'₂, Hal₁ et q ont les significations indiquées ci-dessus,
produits de formules (IX), (XI), (XIII), (XIV), (XV) et (I₂) qui peuvent être des produits de formule (I) et que, pour obtenir des ou d'autres produits de formule (I), l'on peut soumettre, si désiré et si nécessaire, à l'une ou plusieurs des réactions de transformations suivantes, dans un ordre quelconque :
a) une réaction d'estérification de fonction acide,
b) une réaction de saponification de fonction ester en fonction acide,
c) une réaction de transformation de fonction carboxy libre ou estérifié en radical formyle,
d) une réaction de transformation de la fonction cyano en fonction acide,
e) une réaction d'oxydation de groupement alkylthio en sulfoxyde ou sulfone correspondant,
f) une réaction de réduction de la fonction carboxy libre ou estérifié en fonction alcool,
g) une réaction de transformation de fonction alcoxy en fonction hydroxyle, ou encore de fonction hydroxyle en fonction alcoxy,
h) une réaction d'oxydation de fonction alcool en fonction aldéhyde, acide ou cétone,
i) une réaction de transformation du radical formyle en radical carbamoyle,
j) une réaction de transformation du radical carbamoyle en radical nitrile,
k) une réaction de transformation de radical nitrile en tétrazolyle,
l) une réaction de transformation d'une fonction halogénée en fonction formyle ou carboxy estérifié,
m) une réaction de transformation d'un radical formyle en fonction CH₂-CO₂alk ou CH=CH-CO₂alk dans laquelle alk représente un radical alkyle renfermant de 1 à 4 atomes de carbone, puis le cas échéant, transformation en acide correspondant,
n) une réaction de transformation d'un radical formyle en radical 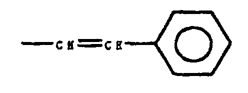 puis le cas échéant en 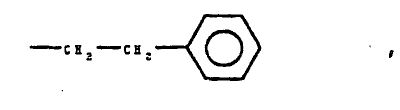
o) une réaction d'oxydation du radical S-alk en 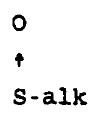 puis transformation en fonction SH et le cas échéant en S-A' dans lequel alk et A' ont la signification indiquée précédemment,
p) une réaction d'élimination des groupements protecteurs que peuvent porter les fonctions réactives protégées,
q) une réaction de salification par un acide minéral ou organique ou par une base pour obtenir le sel correspondant,
r) une réaction de dédoublement des formes racémiques en produits dédoublés,
lesdits produits de formule (I) ainsi obtenus étant sous toutes les formes isomères possibles racémiques, énantiomères et diastéréoisomères.

On peut noter que de telles réactions de transformation de substituants en d'autres substituants peuvent également être effectuées sur les produits de départ ainsi que sur les intermédiaires tels que définis ci-dessus avant de poursuivre la synthèse selon les réactions indiquées dans le procédé décrit ci-dessus.

Dans des conditions préférentielles de mise en oeuvre de l'invention, le procédé décrit ci-dessus peut-être réalisé de la façon suivante. Dans le produit de formule (III), l'atome d'halogène représente de préférence un atome de brome mais peut également représenter un atome de chlore ou d'iode. La réaction de condensation des imidazoles de formules telles que définies ci-dessus (II), (VI), (XVI), (XIII) et (XIV) (dans le cas des produits de formules (XIII) et (XIV), lorsque W représente P et après déprotection de l'atome d'azote), avec le composé de formule (III) telle que définie ci-dessus, pour obtenir respectivement les produits de formules (IV), (VII) lorsque W représente Y', (XV) et (I₁) telles que définies ci-dessus peut être réalisée dans un solvant tel que par exemple le diméthylformamide ou encore le diméthylacétamide, le tétrahydrofuranne, le diméthoxyéthane ou le diméthylsulfoxyde au reflux du solvant ou à la température ambiante, de préférence sous agitation ; la réaction est réalisée de préférence en présence d'une base telle que par exemple l'hydrure de sodium ou de potassium ou encore du carbonate de sodium ou de potassium, du méthylate ou éthylate ou tert-butylate de sodium ou de potassium.

La réaction d'halogénation du composé de formule (IV) telle que définie ci-dessus en composé de formule (V) telle que définie ci-dessus, peut être réalisée dans les conditions usuelles de l'homme du métier et notamment par bromation à l'aide de NBS dans CH₂Cl₂ ou encore Br₂ dans l'acide acétique.

Les composés de formules (V), (VII) et (IX) telles que définies ci-dessus peuvent être soumis à une réaction d'échange halogène métal sur l'atome d'halogène par réaction avec un composé organo métallique tel que le nBuli ou le bromure d'éthyle magnésium dans un solvant tel que le tétrahydrofuranne à une température d'environ -78°C pour le Buli et température ambiante pour le bromure d'éthyle magnésium.

La réaction de carboxylation par CO₂ et de formylation par le diméthylformamide des composés de formules (V) ou (IX) en composé de formule (XI) peut être réalisée selon les conditions usuelles de l'homme de métier soit par exemple dans le tétrahydrofuranne à température ambiante.

L₁ représente un radical alkyle de telle manière que R_{1''} représente les valeurs correspondantes choisies parmi les valeurs de R₁ telles que définies ci-dessus dans lesquelles les éventuelles fonctions réactives sont éventuellement protégées par des groupements protecteurs.

La réaction du composé de formule (V) ou (IX) telles que définies ci-dessus avec le composé de formule (X), telle que définie ci-dessus, pour obtenir le composé correspondant de formule (XI) telle que définie ci-dessus peut être réalisée d'une manière identique en utilisant le bromure d'éthyle magnésium comme agent de métallation dans le tétrahydrofuranne à température ambiante.

La réaction du composé de formule (VII) avec les composés de formules (VIIIa) ou (VIIIb) peut être réalisée selon les conditions usuelles de l'homme de métier soit par exemple dans le tétrahydrofuranne à température ambiante.

La fonction amine des composés de formules (XIII) et (XIV) telles que définies ci-dessus, protégée par P tel que défini ci-dessus, peut être libérée dans les conditions usuelles connues de l'homme du métier et notamment lorsque P représente le radical -CH₂-O-(CH₂)₂-Si(CH₃)₃, l'atome d'hydrogène peut être libéré dans du TFA ou encore en présence d'ion fluorure.

La réaction de saponification peut être réalisée selon les méthodes usuelles connues de l'homme du métier, telles que par exemple dans un solvant tel que le méthanol ou l'éthanol, le dioxane ou le diméthoxyéthane, en présence de soude ou de potasse ou encore de carbonate de cesium.

Les réactions de réduction ou oxydation du produit de formule (XIII) en produit de formules (XIV) peuvent être réalisées selon les méthodes usuelles connues de l'homme du métier.

Dans les réactions décrites ci-dessus, on peut opérer de la façon suivante :
- la réaction du composé de formule (XX) avec le composé de formule (XXI) pour obtenir le composé de formule (XXII) peut être réalisée dans un solvant tel que par exemple le chlorure de méthylène en présence d'un catalyseur acide tel que par exemple l'amberlist puis par réduction par exemple par le borohydrure de sodium dans de l'acide acétique et du chlorure de méthylène,
- la réaction du composé de formule (XXII) avec le composé de formule (XXIII) pour obtenir le composé de formule (XXIV) peut être réalisée dans un solvant tel que par exemple le tétrahydrofuranne ou le chlorure de méthylène, en présence d'une base telle que la pyridine ou encore la triéthylamine ou le carbonate de sodium ou de potassium,
- la réaction du composé de formule (XXIV) avec le composé de formule (XXV) pour obtenir le composé de formule (XXVI) peut être réalisée dans un alcool tel que le méthanol ou l'éthanol en présence d'une base telle que la triéthylamine ou la pyridine.
- la cyclisation du composé de formule (XXVI) en produit de formule (I₂) peut être réalisée en présence d'une anhydride telle que par exemple l'anhydride propane phosphorique dans de l'acétate d'éthyle.

Selon les valeurs de R'₁, R"₁, R"'₁, R'₂, R'₃, R"₃, les produits de formules (IX), (XI), (XIII), (XIV), (XV) et (I₂) constituent ou non des produits de formule (I) et peuvent donner des produits de formule (I), ou être transformés en d'autres produits de formule (I) en étant soumis à une ou plusieurs des réactions a) à r) indiquées ci-dessus.

Ainsi les diverses fonctions réactives que peuvent porter certains composés des réactions définies ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.

La liste suivante, non exhaustive, d'exemples de protection de fonctions réactives peut être citée :
- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle,
- les groupements amino peuvent être protégés par exemple par les radicaux acétyle, trityle, benzyle, tert-butoxycarbonyle, phtalimido ou d'autres radicaux connus dans la chimie des peptides,
- les groupements acyles tel que le groupement formyle peuvent être protégés par exemple sous forme de cétals ou de thiocétals cycliques ou non cycliques tels que le diméthyl ou diéthylcétal ou l'éthylène dioxycétal, ou le diéthylthiocétal ou l'éthylènedithiocétal,
- les fonctions acide des produits décrits ci-dessus peuvent être, si désiré, amidifiées par une amine primaire ou secondaire par exemple dans du chlorure de méthylène en présence, par exemple, de chlorhydrate de 1-éthyl-3-(diméthylaminopropyl) carbodiimide à la température ambiante :
   - les fonctions acide peuvent être protégées par exemple sous forme d'esters formés avec les esters facilement clivables tels que les esters benzyliques ou ter butyliques ou des esters connus dans la chimie des peptides.

Les réactions auxquelles les produits de formules (IX), (XI), (XIII), (XIV), (XV) et (I₂) telles que définies ci-dessus peuvent être soumis, si désiré ou si nécessaire, peuvent être réalisées, par exemple, comme indiqué ci-après.
a) Les produits décrits ci-dessus peuvent, si désiré, faire l'objet, sur les éventuelles fonctions carboxy, de réactions d'estérification qui peuvent être réalisées selon les méthodes usuelles connues de l'homme du métier.
b) Les éventuelles transformations de fonctions ester en fonction acide des produits décrits ci-dessus peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou encore par de l'acide chlorhydrique ou sulfurique.
c) la réaction de transformation sur la fonction ester 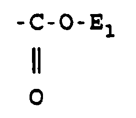 dans laquelle E₁ peut représenter un radical alkyle éventuellement substitué et éventuellement protégé, en fonction formyle peut être réalisée ainsi qu'il est décrit dans la partie expérimentale, ou selon les méthodes usuelles connues de l'homme du métier, notamment par saponification de l'ester en acide, transformé alors en chlorure d'acide par exemple par action du chlorure de thionyle, puis réduction par exemple par hydrogénation sur palladium.
d) Les éventuelles fonctions cyano des produits décrits ci-dessus peuvent être, si désiré, transformées en fonction acide dans les conditions usuelles connues de l'homme du métier par exemple par une double hydrolyse réalisée en milieu acide tel que par exemple dans un mélange d'acide sulfurique, d'acide acétique glacial et d'eau, ces trois composés étant de préférence en proportions égales, ou encore dans un mélange de soude, d'éthanol et d'eau au reflux.
e) Les éventuels groupements alkylthio des produits décrits ci-dessus peuvent être, si désiré, transformés en les fonctions sulfoxyde ou sulfone correspondantes dans les conditions usuelles connues de l'homme du métier telles que par exemple par les peracides comme par exemple l'acide peracétique ou l'acide métachloroperbenzoique ou encore par l'ozone, l'oxone, le périodate de sodium dans un solvant tel que par exemple le chlorure de méthylène ou le dioxanne à la température ambiante.
   L'obtention de la fonction sulfoxyde peut être favorisée par un mélange équimolaire du produit renfermant un groupement alkylthio et du réactif tel que notamment un peracide.
   L'obtention de la fonction sulfone peut être favorisée par un mélange du produit renfermant un groupement alkylthio avec un excès du réactif tel que notamment un peracide.
f) Les éventuelles fonctions carboxy libre ou estérifié des produits décrits ci-dessus peuvent être, si désiré, réduites en fonction alcool par les méthodes connues de l'homme de métier : les éventuelles fonctions carboxy estérifié peuvent être, si désiré, réduites en fonction alcool par les méthodes connues de l'homme du métier et notamment par de l'hydrure de lithium et d'aluminium dans un solvant tel que par exemple le tétrahydrofuranne ou encore le dioxane ou l'éther éthylique.
   Les éventuelles fonctions carboxy libre des produits décrits ci-dessus peuvent être, si désiré, réduites en fonction alcool notamment par de l'hydrure de bore.
g) Les éventuelles fonctions alcoxy telles que notamment méthoxy des produits décrits ci-dessus peuvent être, si désiré, transformées en fonction hydroxyle dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide trifluoro acétique au reflux.
h) Les éventuelles fonctions alcool des produits décrits ci-dessus peuvent être, si désiré, transformées en fonction aldéhyde ou acide par oxydation dans les conditions usuelles connues de l'homme du métier telles que par exemple par action de l'oxyde de manganèse pour obtenir les aldéhydes ou du réactif de Jones pour accéder aux acides.
i) j) Les réactions de transformation de radical formyle en radical carbamoyle et de radical carbamoyle en radical nitrile, sont réalisées notamment pour R₃ et R₄ selon les conditions usuelles connues de l'homme du métier, telles que par exemple passage par le céto nitrile et déplacement par une amine (Chem. Comm. 1971, p. 733).
k) Les éventuelles fonctions nitrile des produits décrits ci-dessus peuvent être, si désiré, transformées en tétrazolyle dans les conditions usuelles connues de l'homme du métier telles que par exemple par cycloaddition d'un azidure métallique tel que par exemple l'azidure de sodium ou un azidure de trialkylétain sur la fonction nitrile ainsi qu'il est indiqué dans la méthode décrite dans l'article référencé comme suit :
   J. Organometallic Chemistry., 33, 337 (1971) KOZIMA S.& coll.

   On peut noter que la réaction de transformation d'un carbamate en urée et notamment d'un sulfonylcarbamate en sulfonylurée, peut être réalisée par exemple au reflux d'un solvant comme par exemple le toluène en présence de l'amine adéquate.
l) la transformation d'un radical halogéné en radical formyle peut être notamment effectuée par action d'un dérivé organo métallique, par exemple le bromure d'éthyl magnésium, au sein d'un solvant organique,
m) la transformation du radical formyle en radical CH=CH-CO₂alk peut être effectuée par une réaction de type wittig par condensation d'un sel de phosphonium approprié en présence d'hydrure de sodium ; la transformation en acide est effectuée par hydrolyse, par exemple au moyen d'une base telle que la soude en milieu alcoolique
n) la transformation du radical formyle en radical 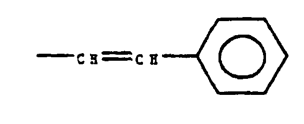 peut être effectuée par réaction de Wittig comme indiqué ci-dessus ; la transformation en radical 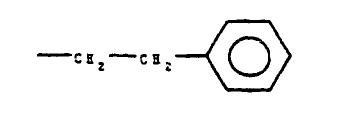 est effectuée par réduction, au moyen d'hydrogène en présence d'un catalyseur, par exemple l'oxyde de platine,
   On peut noter que la transformation du radical formyle en radical CH₂OH peut être réalisée au moyen d'un agent réducteur, par exemple le borohydrure de sodium dans l'éthanol à la température ambiante ; la transformation en radical -CH₂-SR peut être effectuée par action du thiol approprié R-SH sur le mésylate intermédiaire préparé au préalable par action du chlorure de mésyle sur l'alcool en présence de la base de Hunig,
o) l'oxydation du substituant S-alk en sulfoxyde peut être effectuée par exemple, par action de l'acide métachloroperbenzoique ; la transformation du thiol est obtenue par la réaction de PUMMERER par exemple en présence d'anhydride trifluoroacétique ; la transformation du substituant SH en SZ₂ peut être obtenue par action d'un dérivé halogéné Hal-Z₂ par exemple l'iodocyclohexane.
   Il est entendu que les réactions décrites ci-dessus peuvent être effectuées selon les méthodes usuelles connues de l'homme du métier.
p) L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.
   Le groupement phtalimido peut être éliminé par l'hydrazine.
   On trouvera une liste de différents groupements protecteurs utilisables par exemple dans le brevet BF 2 499 995.
q) Les produits décrits ci-dessus peuvent, si désiré, faire l'objet de réactions de salification par exemple par un acide minéral ou organique ou par une base minérale ou organique selon les méthodes usuelles connues de l'homme du métier.
r) Les éventuelles formes optiquement actives des produits décrits ci-dessus peuvent être préparées par dédoublement des racémiques selon les méthodes usuelles connues de l'homme du métier.

Des illustrations de telles réactions définies ci-dessus sont données dans la préparation des exemples décrits ci-après.

Les composés de formule (I) tels que définis ci-dessus ainsi que leurs sels d'addition avec les acides présentent d'intéressantes propriétés pharmacologiques.

Les produits de formule (I) tels que définis ci-dessus, sont doués de propriétés antagonistes pour les récepteurs à l'endothéline et sont ainsi notamment inhibiteurs des effets de l'endothéline, notamment des effets vasoconstricteurs et hypertenseurs induits par l'endothéline. On note en particulier un effet antiischémique, l'activité vasoconstrictrice de l'endothéline étant abolie.

Les produits de formule (I) sont également capables de s'opposer aux effets stimulants de l'endothéline au niveau de tous les types cellulaires, notamment les cellules musculaires lisses, les fibroblastes, les cellules neuronales et les cellules osseuses.

Ces propriétés justifient leur application en thérapeutique et l'invention a particulièrement pour objet à titre de médicaments, les produits de formule (I),
lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I).

L'invention a ainsi plus particulièrement pour objet à titre de médicaments, les produits tels que définis par la formule (I) ci-dessus, dans laquelle :
R₁ représente un radical éthyle, n-propyle, isopropyle, n-butyle, isobutyle ou terbutyle,
A représente un atome de soufre,
R₂ représente un radical alkyle ou alcoxy, linéaires ou ramifiés, renfermant au plus 4 atomes de carbone et substitués par un radical carboxy libre, salifié ou estérifié,
R₃, représente un radical carboxy libre, salifié ou estérifié, ou un radical tétrazolyle libre ou salifié,
Y représente un radical phényle substitué par un radical dioxol et éventuellement par un atome d'halogène,
lesdits produits de formule (I) étant sous toutes les formes isoméres possibles racémiques ou optiquement actives, ainsi que les sels d'addition avec les acides minéraux et organiques ou avec les bases minérales et organiques pharmaceutiquement acceptables desdits produits de formule (I).

L'invention a tout particulièrement pour objet, à titre de médicaments, les produits décrits ci-après dans les exemples et notamment les produits de formule (I) telle que définie ci-dessus, dont les noms suivent :
- acide 4-((4-(carboxyméthoxy) phényl) thio)-1-((6-chloro-1,3-benzodioxol-5-yl) méthyl)-2-propyl-1H-imidazole-5-carboxylique,
- acide 4-((4-(2-carboxyéthyl) phényl) thio)-1-((6-chloro-1,3-benzodioxol-5-yl) méthyl)-2-(1-méthyléthyl)-1H-imidazole-5-carboxylique,
- acide 4-((4-carboxyphényl) thio) 1-((6-chloro 1,3-benzodioxol-5-yl) méthyl) 2-propyl 1H-imidazole 5-carboxylique, ainsi que leurs sels d'addition avec les acides minéraux ou organiques ou avec les bases minérales et organiques pharmaceutiquement acceptables.

Les médicaments, objet de l'invention, trouvent, par exemple, leur emploi dans le traitement de tous les spasmes vasculaires, dans le traitement du vasospasme consécutif à une hémorragie cérébrale, dans le traitement des spasmes coronariens, des spasmes vasculaires périphériques ainsi que dans le traitement des insuffisances rénales. Ces médicaments peuvent également être utilisés dans le traitement de l'infarctus du myocarde, de l'insuffisance cardiaque congestive, dans la prévention des resténoses post-angioplastie, des fibroses cardiaques et vasculaires, dans le traitement de l'athérosclérose, de certaines formes d'hypertension comme notamment l'hypertension pulmonaire, ainsi que dans le traitement de l'asthme.

Les médicaments, objet de l'invention, peuvent également trouver une application dans le traitement de l'ostéoporose, de l'hyperplasie prostatique et en tant que protecteurs neuronaux.

L'invention s'étend aux compositions pharmaceutiques contenant à titre de principe actif l'un au moins des médicaments tels que définis ci-dessus.

Ces compositions pharmaceutiques peuvent être administrées par voie buccale, rectale, par voie parentérale ou par voie locale en application topique sur la peau et les muqueuses ou par injection par voie intraveineuse ou intramusculaire.

Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 1 à 300 mg par jour chez l'adulte, par voie orale ou de 1 à 100 mg par jour par voie intraveineuse.

Certains produits de départ de formules (II) et (XVI) sont connus et peuvent être préparés par exemple comme indiqué dans le brevet européen EP 168 950.

D'autres produits de départ de formules (II) et (XVI) peuvent en particulier être préparés comme indiqué dans le brevet européen EP 0465368, ou encore dans les exemples décrits ci-après dans la partie expérimentale.

Certains produits de départ de formules (II) et (XVI) sont commerciaux tels que par exemple, les produits de formule (II) suivants :
- le 2-propylimidazole, le 2-isopropylimidazole, le 2-éthylimidazole, le 2-méthylimidazole.

Des exemples de produits commerciaux de formule (XVI) sont donnés dans les brevets EP 0465368 ou EP 0503162.

On peut encore notamment préparer certains produits de formules (II) à partir d'autres produits de formule (II) par exemple en les soumettant à une ou plusieurs des réactions décrites ci-dessus en a) à r), réalisées dans les conditions également décrites ci-dessus.

Les composés de départ de formule (VI) tel que le 2,4,5-tribromoimidazole ou encore les produits de départ de formules (XX) et (XXI) peuvent être disponibles dans le commerce ou peuvent être préparés selon les méthodes usuelles connues de l'homme du métier.

Les produits de départ de formule (X), sont commerciaux tels que notamment :
- le méthyl chloroformate
- le benzyl chloroformate
- l'isobutyl chloroformate
- l'éthyl chloroformate
- le N-propyl chloroformate.

Les produits de départ de formules (VIIIa) et (VIIIb) . sont commerciaux tels que notamment :
les produits de formule (VIIIa) suivants :
   - le benzaldehyde ou butanal
les produits de formule (VIIIb) suivants :
   - le chlorure de benzoyle ou de butyryle.

Un procédé de préparation de certains produits de formule (III) est notamment décrit dans le brevet européen EP 0465368.

Des exemples de préparation de composés de formule (III) sont également décrits dans la littérature et des exemples en sont donnés notamment dans le brevet US 4,880,804 ou par exemple dans la référence Chemistry and Industry 7 september 1987 HOWARD and COLQUHOUN pp. 612-617.

Notamment, le produit de formule (III) qui est le chlorure de 6-chloro pipéronyle est commercial chez ACROS.

La présente invention a enfin pour objet à titre de produits industriels nouveaux, les composés de formules (IV), (V), (IX), (XI) et (XIII), dans lesquels, étant entendu que dans les composés de formules (IX) et (XI), W représente le radical CH₂-Y', Y' représente le radical phényle substitué par un radical dioxol et éventuellement par un atome d'halogène ou par un radical alkyle ou alcoxy renfermant au plus 4 atomes de carbone et dans lesquelles les éventuelles fonctions réactives sont éventuellement protégées par des groupements protecteurs.

L'invention a ainsi particulièrement pour objet l'utilisation des produits de formule (I) telle que définie ci-dessus, pour la préparation de compositions pharmaceutiques destinées au traitement d'affections résultant d'une stimulation anormale des récepteurs de l'endothéline et notamment destinées au traitement de l'hypertension induite par l'endothéline, de tous les spasmes vasculaires, au traitement des suites d'une hémorragie cérébrale, des insuffisances rénales, de l'infarctus du myocarde, de l'insuffisance cardiaque et à la prévention des resténoses post-angioplastie ainsi que des fibroses cardiaques et vasculaires.

Les exemples suivants illustrent l'invention sans toutefois la limiter.

### EXEMPLE 1 : acide 4-((4 -(carboxyméthoxy) phényl) thio)-1-((6-chloro-1,3-benzodioxol 5-yl) méthyl) 2-propyl 1H-imidazole 5-carboxylique

### STADE 1 : 1-((6-chloro 1,3-benzodioxol-5-yl) méthyl)-2-n-propyl 1H-imidazole

On introduit 12 g de 2-n-propyl-1H-imidazole dans 125 ml de diméthylformamide anhydre, ajoute lentement par fraction 5,28 g d'hydrure de sodium à 50 % dans l'huile, agite pendant 20 minutes puis introduit par fractions 22,55 g de chlorure de 6-chloro-pipéronyl et agite une heure à température ambiante. On évapore le diméthylformamide au rotavapor puis le résidu est acidifié puis hydrolyse par du chlorure d'ammonium saturé. On extrait 3 fois par du chlorure de méthylène. La phase organique est lavée avec de l'eau distillée, séchée, puis empâtée dans de l'éther éthylique et séchée.

On obtient ainsi 22,2 g de produit attendu (poudre blanche).

### Contrôle :

IR CHCl₃ cm⁻¹
Absence de N-H
Hétérocycle et aromatique : 1627, 1575, 1523, 1506, 1483.

### STADE 2 : 1-((6-chloro-1,3-benzodioxol 5-yl)-méthyl)-4,5-dibromo-2-n-propyl-1H-imidazole

On introduit 25 g du produit obtenu au stade 1 ci-dessus, dans 150 ml de chlorure de méthylène anhydre, ajoute par fractions 35,2 g de N-bromo succinimide et porte le mélange réactionnel à reflux de chlorure de méthylène pendant 2 heures. La solution est lavée avec de la soude 1M puis la phase organique est lavée avec de l'eau distillée puis du chlorure de sodium saturé. La phase organique est séchée, filtrée, concentrée, empâtée dans de l'éther éthylique et séchée. On obtient ainsi 34,87 g de produit attendu (poudre jaune).

### Contrôle :

IR CHCl₃ cm⁻¹
Hétérocycle et aromatique : 1627, 1506, 1484.

### STADE 3 : 1-((6-chloro-1,3-benzodioxol-5-yl) méthyl)-4-bromo 2-n-propyl-1H-imidazole-5-carboxaldéhyde

On introduit 20 g du produit obtenu au stade 2 ci-dessus, dans 100 ml de tétrahydrofuranne anhydre. On ajoute 30,6 ml de solution à 3M de bromure d'éthyle magnésien dans le diéthyl éther, agite 20 minutes à température ambiante puis on introduit 35,6 ml de diméthylformamide et agite encore 2 heures. La solution est acidifiée, hydrolysée par du chlorure d'ammonium, saturée puis extraite par de l'acétate d'éthyle. La phase organique est lavée par de l'eau distillée puis par du chlorure de sodium saturé. La phase organique est séchée, filtrée et concentrée. Pour purifier, on chromatographie sur silice avec comme éluant : acétate d'éthyle 2-cyclohexane 8 et séchée. On obtient ainsi 13,11 g de produit attendu (poudre blanche).

### Contrôle :

IR CHCl₃ cm⁻¹
Carbonyle : 1669
Hétérocycle et aromatique : 1506, 1485.

### STADE 4 : 1-((6-chloro-1,3-benzodioxol 5-yl) méthyl)-4-((4-hydroxyphényl) thio)-2-n-propyl 1H-imidazole-5-carboxaldéhyde

On introduit 1,8 g d'hydroxythiophénol, ainsi que 12 ml de tétrahydrofuranne anhydre et 1,76 g d'hydrure de sodium par petites fractions. Après 15 minutes d'agitation à température ambiante, on ajoute 58 ml de diméthylformamide anhydre ainsi que 1,8 g du produit obtenu au stade 3 ci-dessus. Le mélange est agité 1 heure à température ambiante, puis hydrolysé et acidifié par du chlorure de sodium. La phase aqueuse est extraite 2 fois avec 75 ml d'acétate d'éthyle. Les phases organiques, rassemblées, sont lavées à l'eau, puis avec une solution saturée en chlorure de sodium. On sèche, filtre, concentre, empâte dans l'éther éthylique, et obtient 1,72 g de produit attendu (solide de couleur crème). F = 241,5°C.

### Contrôle :

IR Nujol cm⁻¹
Absorption OH/NH
>= O ~ 1660
Hétérocycle et aromatique : 1594, 1576, 1499, 1484.

### STADE 5 : 1-((6-chloro-1,3-benzodioxol-5-yl) méthyl)-4-((4-(éthoxycarbonylméthoxy) phényl) thio)-2-n-propyl 1H-imidazole 5-carboxaldéhyde

On introduit 0,7 g du produit obtenu au stade 4 ci-dessus, 18 ml de tétrahydrofuranne, 9 ml de diméthylformamide et 153 mg d'hydrure de sodium par petites fractions. Après 10 minutes d'agitation à température ambiante, 0,21 ml de bromoacétate d'éthyle sont rajoutés et l'agitation est maintenue à température ambiante. On ajoute 35 ml d'eau, puis extrait la phase aqueuse à l'acétate d'éthyle. Les phases organiques, rassemblées, sont lavées à l'eau, puis avec une solution saturée en chlorure de sodium. On sèche, filtre, concentre. On chromatographie sur silice, avec pour éluant : 90 % chlorure de méthylène, 10 % acétate d'éthyle.

On obtient ainsi 0,78 g de produit attendu (huile légèrement jaune).

### Contrôle :

IR CHCl₃ cm⁻¹
Absence d'OH
>= O : 1756, 1734, 1663
Hétérocycle et aromatique : 1593, 1575, 1505, 1493, 1484.

### STADE 6 : 1-((6-chloro-1,3-benzodioxol-5-yl) méthyl)-2-n-propyl-1H-imidazole-5-cyano-4-((4-méthoxyphénylthio) carboxylate d'éthyle

On introduit 15 ml d'isopropanol, que l'on sature avec de l'ammoniac gazeux. On dissout le produit obtenu au stade 5 ci-dessus dans 10 ml d'isopropanol, ajoute 1,98 g d'oxyde de manganèse, chauffe au bain-marie pendant environ 15 minutes puis agite environ une nuit à température ambiante. On filtre, concentre et purifie par chromatographie sur silice, avec pour éluant 95 % de chlorure de méthylène, 5 % de méthanol.

On obtient ainsi 400 mg de produit attendu.

### STADE 7 : acide 4-((4-(carboxyméthoxy) phényl) thio)-1-((6-chloro-1,3-benzodioxol-5-yl) méthyl)-2-propyl-1H-imidazole-5-carboxylique

On introduit 400 mg du produit obtenu au stade 6 ci-dessus, dans 25 ml d'éthanol, ajoute 10 ml de soude (5N) et le mélange réactionnel est porté à reflux une nuit.

L'éthanol est évaporé, et on porte à reflux dans 10 ml de soude 5N, pendant 4 heures. On ajoute alors 20 ml d'eau distillée, filtre, ajoute 35 ml d'acide chlorhydrique 2N et le précipité obtenu est essoré, lavé abondamment à l'eau distillée, puis empâté dans l'éthanol. On recristallise dans un mélange éthanol, acétate d'éthyle, chlorure de méthylène (50 %, 25 %, 25 %) et obtient ainsi 125 mg de produit attendu. F = 202°C.

### Contrôle :

IR Nujol cm⁻¹
Absorption générale région OH/NH
C=O : 1735, 1660
Hétérocycle et aromatique : 1593, 1576, 1502, 1493, 1485.

### EXEMPLE 2 : 4-((4-(2-carboxyéthyl) phényl) thio)-1-((6-chloro-1,3-benzodioxol-5-yl) méthyl)-2-(1-méthyléthyl)-1H-imidazole-5-carboxaldéhyde

### STADE 1 : 1-((6-chloro-1,3-benzodioxol-5-yl) méthyl)-2-isopropyl-1H-imidazole

On procède comme au stade 1 de l'exemple 1, en utilisant 22 g du 2-isopropyl 1H-imidazole au lieu du 2-n-propyl-1H-imidazole. On obtient ainsi 27,8 g de produit attendu.

### STADE 2 : 1-((6-chloro-1,3-benzodioxol-5-yl) méthyl)-4,5-dibromo-2-isopropyl-1H-imidazole

On procède comme au stade 2 de l'exemple 1, en utilisant 27,8 g du produit obtenu au stade 1 ci-dessus au lieu du produit obtenu au stade 1 de l'exemple 1. On obtient ainsi 39,2 g de produit attendu.

### STADE 3 : 1-((6-chloro 1,3-benzodioxol-5-yl) méthyl)-4-bromo 2-isopropyl-1H-imidazole-5-carboxaldéhyde

On procède comme au stade 3 de l'exemple 1, en utilisant 25 g du produit obtenu au stade 2 ci-dessus, au lieu du produit obtenu au stade 2 de l'exemple 1.

On obtient ainsi 11,8 g de produit attendu.

### STADE 4 : 4-((4-(2-carboxyéthyl) phényl) thio) 1-((6-chloro 1,3-benzodioxol 5-yl) méthyl) 2-(1-méthyléthyl) 1H-imidazole 5-carboxaldéhyde

On opère comme au stade 4 de l'exemple 1, à partir de 770 mg du produit obtenu au stade 3 ci-dessus, au lieu du produit obtenu au stade 3 de l'exemple 1 et de 550 mg de N3 pour obtenir 610 mg de produit attendu. Solide amorphe.

### EXEMPLE 3 : acide 4-((4-(2-carboxyéthyl) phényl) thio)-1-((6-chloro-1,3-benzodiol-5-yl) méthyl)-2-(1-méthyléthyl)-1H-imidazole-5-carboxylique

### STADE 1 : Acide 3-[4-[[1-[(6-chloro-1,3-benzodioxol-5-yl) méthyl]-5-cyano-2-(1-méthyléthyl)-1H-imidazol-4-yl]thio]phénoxy]-propanoïque

On procède comme au stade 6 de l'exemple 1 à partir de 600 mg du produit de l'exemple 2, et obtient ainsi 200 mg de produit attendu.

### STADE 2 : acide 4-((4-(2-carboxyéthyl) phényl) thio)-1-((6-chloro-1,3-benzodioxol 5-yl) méthyl)-2-(1-méthyléthyl)-1H-imidazole-5-carboxylique

On procède comme au stade 7 de l'exemple 1 à partir de 200 mg du produit obtenu au stade 1 ci-dessus et obtient ainsi 115 mg de produit attendu. F = 198°C.

### EXEMPLE 4 : acide 4-((4-carboxyméthyl) 2,3,5,6-tétrafluorophényl) thio) 1-((6-chloro 1,3-benzodioxol-5-yl) méthyl) 2-propyl 1H-imidazole 5-carboxylique.

### STADE 1 : cyano-[(1-oxobutyl) amino] acétate d'éthyle

On mélange 5 g d'éthyl (hydroxyimino) cyanoacétate, 40 cm³ de tétrahydrofuranne, 11,5 cm³ d'anhydride butyrique et 2,5 g de platine et agite en atmosphère d'hydrogène jusqu'à saturation. On filtre, rince par 5 x 15 cm³ d'éther éthylique, évapore l'éther, ajoute peu à peu 200 cm³ d'essence G, essore, lave avec 3 x 10 cm³ d'essence G et sèche à environ 75°C. On concentre à ~ 10 cm³, ajoute 50 cm³ d'essence G, laisse cristalliser 30 mn à température ambiante, essore, lave avec 3 x 3 cm³ d'essence G et sèche à environ 75°C. On obtient 5,73 g de produit. F = 110°C.

### Recristallisation pour analyses :

On dissout 540 mg du produit obtenu dans 50 cm³ d'éther isopropylique à reflux, filtre, concentre, laisse environ 1 h au repos à température ambiante, essore, lave à l'éther isopropylique et sèche. On obtient 440 mg de produit attendu. F = 110°C.

### Stade 2 : 3-amino 2-[(1-oxobutyl) amino] 3-(méthylthio) 2-propenoate d'éthyle

A une solution de 20 g de nitrile obtenu au stade A ci-dessus, dans 400 ml d'éthanol, on ajoute 1,4 ml de triéthylamine, on refroidit à environ -10°C et introduit environ 22 g de méthylmercaptan par barbotage. On agite environ 72 heures à 0°C. On élimine l'excès de méthanethiol chasse l'éthanol, empâte à l'essence G, filtre et sèche. On obtient 24,3 g de produit attendu (cristaux incolores).
F_{K115} = 120-124°C

### Stade 3 : 4-(méthylthio) 2-propyl 1H-imidazole 5-carboxylate d'éthyle

A 20,1 g de pentachlorure de phosphore dans 300 cm³ de chlorure de méthylène, refroidis à environ -70°C on ajoute une solution de 12,9 g de 4-diméthylaminopyridine dans 90 cm³ de chlorure de méthylène. On maintient encore environ 15 mn à environ -70°C puis on introduit une solution de 12 g du produit obtenu au stade B ci-dessus dans 120 cm³ de chlorure de méthylène. On laisse revenir à température ambiante et maintient sous agitation pendant environ 22 heures. On verse le mélange réactionnel dans 2,5 l d'eau + glace et neutralise par addition d'environ 60 g de bicarbonate de sodium. On agite encore environ 30 mn, on décante et on extrait avec 500 cm³ de chlorure de méthylène. On lave à l'eau salée, sèche et chasse le solvant à environ 50°C. On purifie par chromatographie sur silice avec pour éluants chlorure de méthylène-acétate d'éthyle (90-10) puis chlorure de méthylène-acétate d'éthyle (80-20). On chasse les solvants à environ 50°C, empâte à l'essence G, filtre et sèche. On obtient 7,4 g de produit attendu (cristaux incolores). F_{K95} = 85°C.

### STADE 4 : 1-[(6-chloro-1,3-benzodioxol-5-yl)méthyl]-4-(méthylthio)-2-propyl-1H-imidazole-5-carboxylate d'éthyle

On introduit 10 g du produit obtenu au stade 3 ci-dessus et 13,5 g de chlorure de 6-chloro pipéronyl dans 100 ml de diméthylformamide, ajoute 9 g de carbonate de potassium et chauffe à 100°C pendant une heure. On refroidit à température ambiante, puis verse le milieu réactionnel sur de la glace, filtre le précipité obtenu puis l'empâte avec de l'éther isopropylique. On obtient ainsi 9,25 g de produit attendu.

### STADE 5 : 1-[(6-chloro-1,3-benzodioxol-5-yl)méthyl]-4-(méthylsulfinyl)-2-propyl-1H-imidazole-5-carboxylate d'éthyle

On introduit 9,26 g du produit obtenu au stade 4 ci-dessus dans 400 ml de chlorure de méthylène à 0°C et ajoute 5,8 g d'acide métachloroperbenzoïque. On agite une heure à température ambiante et lave le milieu réactionnel avec de l'hydrure de sodium et de potassium, extrait au chlorure de méthylène, lave à l'eau, sèche, évapore le solvant. On obtient après chromatographie 8,8 g de produit attendu.
F = 187°C.

### STADE 6 : 1-[(6-chloro-1,3-benzodioxol-5-yl)méthyl]-4-mercapto-2-propyl-1H-imidazole-5-carboxylate d'éthyle

On introduit 800 mg du produit obtenu au stade 5 ci-dessus dans 16 ml de chlorure de méthylène et on ajoute 0,54 ml d'anhydride trifluoroacétique. On agite pendant 30 minutes, évapore à sec et reprend le résidu dans 10 ml de méthanol et 4 ml de triéthylamine. On agite 30 minutes, extrait au chloroforme, lave avec une solution saturée de chlorure d'ammonium, sèche et évapore à sec. On obtient ainsi 720 mg de produit attendu.

### STADE 7 : 1-[(6-chloro-1,3-benzodioxol-5-yl)méthyl]-4-[[(4-carboxyméthyl) 2,3,5,6-tétrafluorophényl] thiol 2-propyl 1H-imidazole 5-carboxylate d'éthyle.

On introduit 1 g du produit obtenu au stade 6 ci-dessus dans 50 ml de tétrahydrofuranne, ajoute 130 mg d'une suspension d'hydrure de sodium à 60 % dans l'huile et laisse agiter 15 minutes à température ambiante. On ajoute ensuite 830 mg de pentafluorophényl acétate de méthyle en solution dans 20 ml de diméthylformamide, et laisse agiter 15 h à température ambiante. On hydrolyse par addition d'acide chlorhydrique dilué et extrait à l'acétate d'éthyle. On obtient ainsi après chromatographie sur silice (éluant : cyclohexane-AcOEt 80-20) 900 mg de produit attendu.

### STADE 8 : acide 4-((4-carboxyméthyl) 2,3,5,6-tétrafluorophényl) thio) 1-((6-chloro 1,3-benzodioxol-5-yl) méthyl) 2-propyl 1H-imidazole 5-carboxylique.

On agite pendant 72 heures 900 mg de produit obtenu au stade 7 dans 50 ml d'éthanol en présence de 20 ml de soude 2N. On évapore le solvant sous pression réduite, reprend le résidu dans l'eau, filtre, acidifie par addition d'acide chlorhydrique N, essore le précipité, le lave à l'eau, le sèche à 50°C sous pression réduite, le purifie par empâtage dans l'éther isopropylique et recueille 680 mg de produit attendu. F = 132°C.

En opérant comme aux stades 4 à 7 de l'exemple 1 en utilisant au départ le composé obtenu au stade 3 et en remplaçant l'hydroxythiophénol par le 4-thiobenzoate de méthyle (J. Org. Chem. 1975, 40 (12) 1745-1748), on a préparé le produit suivant :

### EXEMPLE 5 : acide 4-((4-carboxyphényl) thio) 1-((6-chloro 1,3-benzodioxol-5-yl) méthyl) 2-propyl 1H-imidazole 5-carboxylique.

F = 210°C.

### EXEMPLE 6 : COMPOSITION PHARMACEUTIQUE :

On a préparé des comprimés répondant à la formule suivante :

| | |
|---|---|
| Produit de l'exemple 1 | 50 mg |
| Excipient pour un comprimé terminé à | 200 mg |
| (détail de l'excipient : lactose, talc, amidon, stéarate de magnésium). | |

### RESULTATS PHARMACOLOGIQUES

### 1) ETUDE DE L'AFFINITE POUR LE RECEPTEUR A DE L'ENDOTHELINE

On effectue une préparation membranaire à partir de coeur (ventricules) de rat. Le tissu est broyé au POLYTRON dans un tampon Tris 50 mM pH = 7,4.

Après 30 minutes à 25 °C (B.M.) l'homogénat est centrifugé à 30000 g pendant 15 minutes (2 centrifugations avec reprise intermédiaire dans le tampon Tris pH. 7,4).

Les culots sont remis en suspension dans un tampon d'incubation (Tris 25 mM, pepstatine A 5 microg/ml, aprotinine 3 microg/ml, PMSF 0,1 mM, EDTA 3mM, EGTA 1mM pH 7,4).

On répartit des fractions aliquotes de 2 ml dans des tubes à hémolyse et ajoute de la 125I Endothéline (environ 50000 dpm/tube) et le produit à étudier. (Le produit est d'abord testé à 3*10⁻⁵ M en triple). Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %.

La liaison non spécifique est déterminée par addition d'endothéline à 10⁻⁶ M (en triple). On incube à 25 °C pendant 60 minutes, remet au bain-marie à 0 °C, pendant 5 minutes, filtre sous pression réduite, rince au tampon Tris 7,4 et compte la radioactivité en présence du scintillant Triton.

Le résultat est exprimé directement en concentration inhibitrice 50 % (CI50), c'est-à-dire en concentration de produit étudié exprimée en nM, nécessaire pour déplacer 50 % de la radioactivité spécifique fixée sur le récepteur étudié.

### Résultat :

Les CI50 trouvées pour les produits des exemples sont données dans le tableau I ci-après, en nanomoles.

**TABLEAU I**

| Produit des exemples | Récepteur A de l'endothéline CI50 en nanomoles |
|---|---|
| 1 | 2,5 |
| 3 | 2,5 |

### 2) ETUDE DE L'AFFINITE POUR LE RECEPTEUR B DE L'ENDOTHELINE

On effectue une préparation membranaire à partir de cortex postérieur plus cervelet de rat. Le tissu est broyé au POLYTRON dans un tampon Tris 50 mM pH = 7,4.
Après 30 minutes à 25 °C (B.M.) l'homogénat est centrifugé à 30000 g pendant 15 minutes (2 centrifugations avec reprise intermediaire dans le tampon Tris pH. 7,4).
Les culots sont remis en suspension dans un tampon d'incubation (Tris 25 mM, pepstatine A 5 microg/ml, aprotinine 3 microg/ml, PMSF 0,1 mM, EDTA 3mM, EGTA 1mM pH 7,4).
On répartit des fractions aliquotes de 2 ml dans des tubes à hémolyse et ajoute de la 125I Endothéline (environ 50000 dpm/tube) et le produit à étudier. (Le produit est d'abord testé à 3*10⁻⁵ M en triple). Lorsque le produit testé déplace de plus de 50 % la radioactivité liée spécifiquement au récepteur, il est testé à nouveau selon une gamme de 7 concentrations afin de déterminer la concentration qui inhibe de 50 **%** la radioactivité liée spécifiquement au récepteur. on détermine ainsi la concentration inhibitrice 50 %.
La liaison non spécifique est déterminée par addition d'endothéline à 10⁻⁶ M (en triple). On incube à 25 °C pendant 60 minutes, remet au bain-marie à 0 °C, pendant 5 minutes, filtre sous pression réduite, rince au tampon Tris 7,4 et compte la radioactivité en présence du scintillant Triton.
Le résultat est exprimé directement en concentration inhibitrice 50 % (CI50), c'est-à-dire en concentration de produit étudié exprimée en nM, nécessaire pour déplacer 50 % de la radioactivité spécifique fixée sur le récepteur étudié.
Les CI50 trouvées pour les produits des exemples sont données dans le tableau I ci-après, en nanomoles.

### Résultats :

**TABLEAU I**

| Produit des exemples | Récepteur B de l'endothéline CI₅₀ en nanomoles |
|---|---|
| 1 | 88,5 |
| 3 | 78,4 |

### 3) Test d'activité antagoniste de l'endothéline chez le rat démédullé

Des rats mâles Sprague-Dawley (250 à 350 g) sont anesthésiés (pentobarbital sodique 60 mg/kg injecté par voie intrapéritonale). L'animal est placé sous respiration assistée, une section bilatérale des nerfs vagues est effectuée. Le rat est ensuite démédullé.

La pression artérielle moyenne est enregistrée grâce à un cathéter (PE50) hépariné placé dans la carotide de l'animal et relié à un enregistreur par l'intermédiaire d'un capteur de pression et d'un amplificateur (pressure processor Gould). Un cathéter est implanté dans la veine pudendale afin de permettre l'injection des molécules à étudier. Après une période de stabilisation (15 mn environ), le produit ou le solvant est injecté 10 minutes avant une gamme de doses croissantes d'endothéline injectées toutes les 2 minutes (0.1-0.3-1-3-10 µg/kg).

L'activité antagoniste des produits est estimée par le pourcentage d'inhibition de l'augmentation de pression induite par l'endothéline aux doses de 3 et 10 µg/kg.

### Résultats

| EXEMPLES | Doses mg/kg | % inhibition de vasoconstriction | |
|---|---|---|---|
| | | Et₁ 3 µg/kg | Et₁ 10 µg/kg |
| 1 | 10 | -52 | -50 |
| 3 | 10 | + 7 | -31 |

## Revendications

1. Produits de formule (I) : 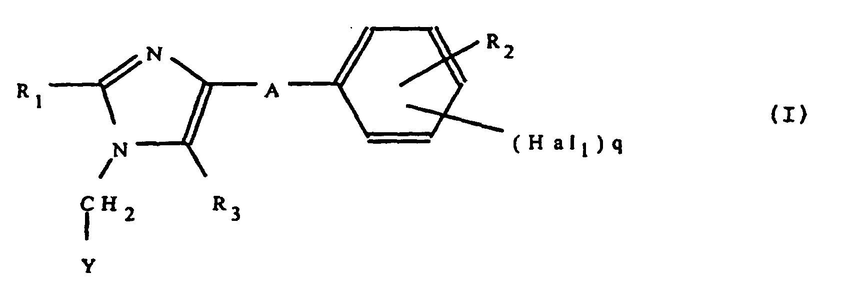 dans laquelle :
R₁ représente un radical éthyle, n-propyle, isopropyle, n-butyle, isobutyle ou terbutyle,
A représente un atome de soufre,
R₂ représente un radical alkyle ou alcoxy, linéaires ou ramifiés, renfermant au plus 4 atomes de carbone et substitués par un radical carboxy libre, salifié ou estérifié,
q est égal à 0,
R₃, représente un radical carboxy libre, salifié ou estérifié, ou un radical tétrazolyle libre ou salifié,
Y représente un radical phényle substitué par un radical dioxol et éventuellement par un atome d'halogène,
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

2. Les produits suivants :
- acide 4-((4-(carboxyméthoxy) phényl) thio)-1-((6-chloro-1,3-benzodioxol-5-yl) méthyl)-2-propyl-1H-imidazole-5-carboxylique,
- acide 4-((4-(2-carboxyéthyl) phényl) thio)-1-((6-chloro-1,3-benzodioxol-5-yl) méthyl)-2-(1-méthyléthyl)-1H-imidazole-5-carboxylique,
- acide 4-((4-carboxyphényl) thio) 1-((6-chloro 1,3-benzodioxol-5-yl) méthyl) 2-propyl 1H-imidazole 5-carboxylique.

3. Procédé de préparation des produits de formule (I), telle que définie à la revendication 1, **caractérisé en ce que** :
soit l'on soumet un composé de formule (II) : 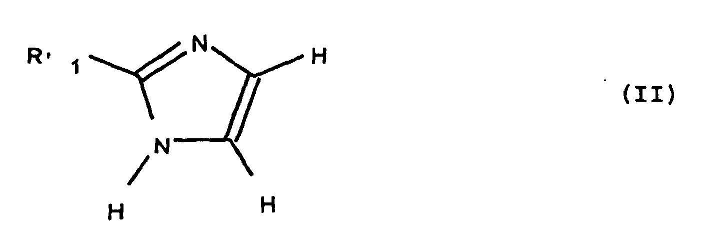 dans laquelle R'₁ a la signification indiquée à la revendication 1 pour R₁, dans laquelle les éventuelles fonctions réactives sont éventuellement protégées par des groupements protecteurs,
à une réaction avec un composé de formule (III) : 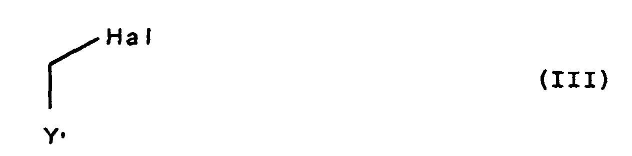 dans laquelle Hal représente un atome d'halogène, et
Y' a la signification indiquée à la revendication 1 pour Y, dans laquelle les éventuelles fonctions réactives sont éventuellement protégées par des groupements protecteurs, pour obtenir le produit de formule (IV) : 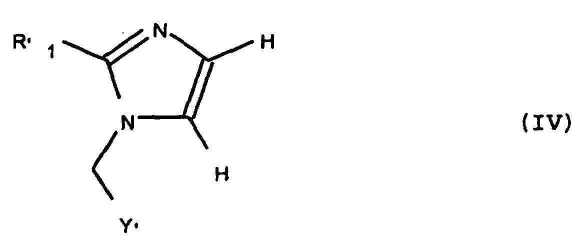 dans laquelle R'₁ et Y' ont les significations indiquées ci-dessus; produit de formule (IV) que l'on peut soumettre à une réaction d'halogénation, pour obtenir le produit de formule (V) : 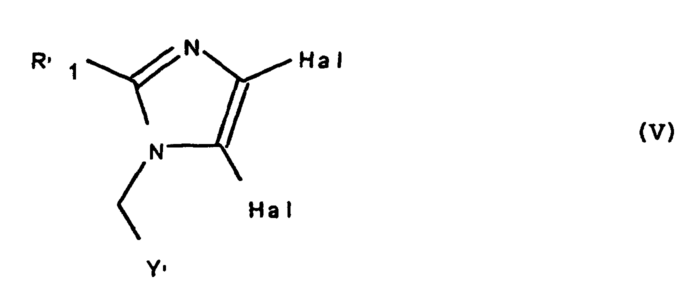 dans laquelle R'₁, Hal et Y' ont les significations indiquées ci-dessus,
soit l'on soumet un composé de formule (VI) : 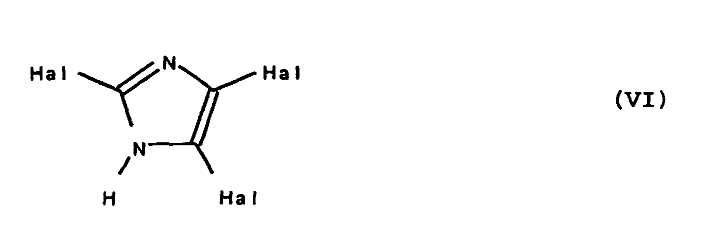
dans laquelle Hal a la signification indiquée ci-dessus,
soit à une réaction avec le composé de formule (III) telle que définie ci-dessus, soit à l'action d'un groupement protecteur P, pour obtenir un produit de formule (VII) : 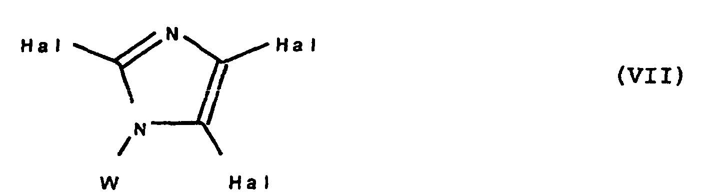 dans laquelle Hal a la signification indiquée ci-dessus et W représente soit -CH₂-Y' avec Y' tel que défini ci-dessus, soit P qui représente un groupement protecteur de l'atome d'azote, produit de formule (VII) que l'on soumet à une réaction d'échange halogène-métal puis à une réaction avec le diméthylformamide ou avec un électrophile de formule (VIIIₐ) ou (VIII_{b}) :
L₁-CHO (VIIIₐ)
L₁-CO-Cl (VIII_{b})
dans lesquelles L₁ représente un radical alkyle, linéaire ou ramifié, renfermant au plus 6 atomes de carbone et éventuellement substitué par un radical alcoxy ou hydroxyle protégé, pour obtenir un produit de formule (IX) : 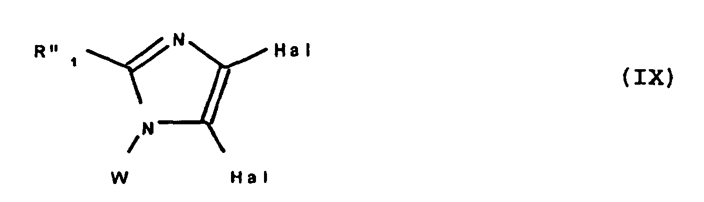 dans laquelle Hal et W ont les significations indiquées ci-dessus, R"₁ représente un radical alkyl-carbonyle, formyle ou hydroxyalkyle dans lesquels le radical alkyle a la signification indiquée ci-dessus et dans lesquels les éventuelles fonctions réactives sont éventuellement protégées par des groupements protecteurs, produits de formules (v) et (IX) que l'on peut soumettre à une réaction d'échange halogène-métal sur l'un des atomes d'halogène puis à une réaction avec CO₂ ou diméthylformamide ou un composé électrophile de formule (X) : 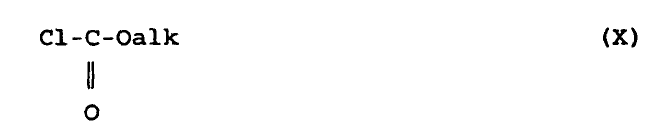 dans laquelle alk représente un radical alkyle renfermant au plus 4 atomes de carbone,
pour obtenir le composé de formule (XI) : 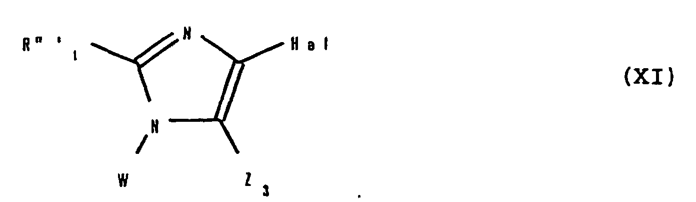 dans laquelle R'' '₁ représente R'₁ ou R"₁ tels que définis ci-dessus, Hal, Y' et W ont les significations indiquées ci-dessus et Z₃ représente le radical carboxy, libre ou estérifié ou le radical formyle,
composé de formule (XI) que l'on peut soumettre à une réaction avec un composé de formule (XII) : 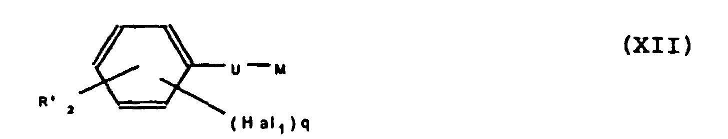 dans laquelle U représente un atome de soufre ou d'oxygène, M représente un métal tel que sodium, potassium ou cuivre, Hal₁ et q ont la signification indiquée à la revendication 1, R'₂ représente R₂ tel que défini à la revendication 1, dans lequel les éventuelles fonctions réactives sont éventuellement protégées par des groupements protecteurs pour obtenir le composé de formule (XIII) : 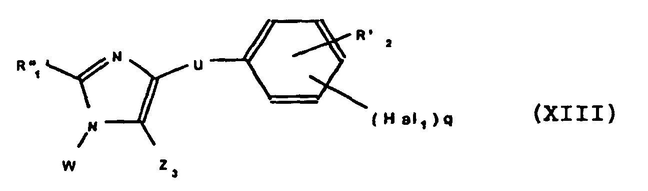 dans laquelle R"'₁, U, R'₂, Hal₁, q et W ont les significations indiquées ci-dessus, produit de formule (XIII) que lorsque Z₃ représente le radical formyle, l'on peut soumettre à une réaction d'oxydation pour obtenir le produit de formule (XIV) : 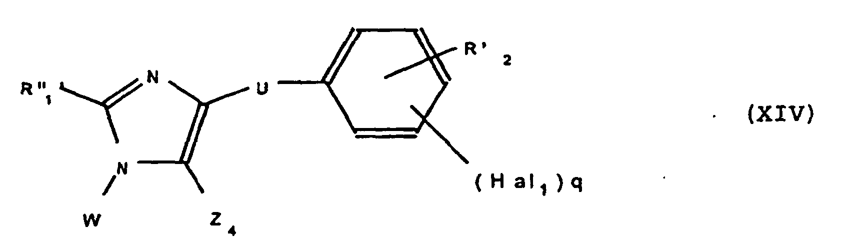 dans lequel R'' '₁, U, R'₂, Hal₁, q et W ont les significations indiquées ci-dessus, et Z₄ représente le radical cyano ou le radical carboxy libre ou estérifié par un radical alkyle linéaire ou ramifié renfermant au plus 6 atomes de carbone, produits de formule (XIII) ou (XIV) que, dans le cas où W représente P tel que défini ci-dessus et après libération de la fonction amine bloquée par P tel que défini ci-dessus, l'on fait réagir avec le composé de formule (III) telle que définie ci-dessus, pour obtenir un produit de formule (XV) : 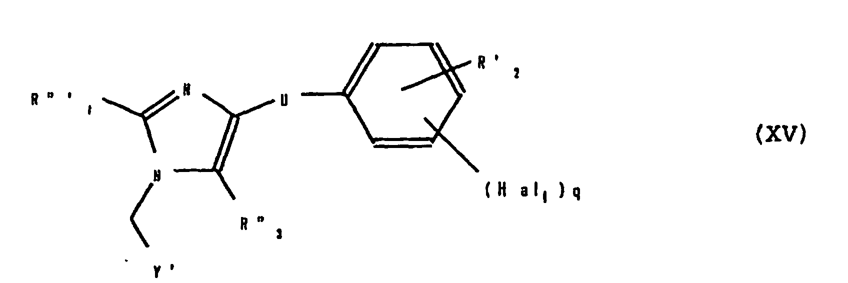 dans laquelle R"'₁, U, R'₂, Hal₁, q et Y' ont les significations indiquées ci-dessus, et R"₃ représente Z₃ ou Z₄ tels que définis ci-dessus,
soit l'on soumet un composé de formule (XX) :
Y'-CHO (XX)
dans laquelle Y' a la signification indiquée ci-dessus, à un composé de formule (XXI) : 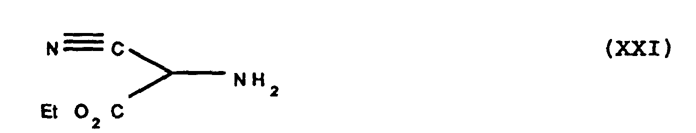 pour obtenir un produit de formule (XXII) : 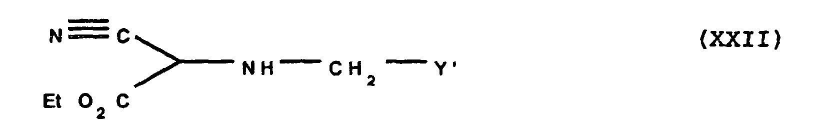 dans laquelle Y' a la signification indiquée ci-dessus, que l'on soumet à un composé de formule (XXIII) : 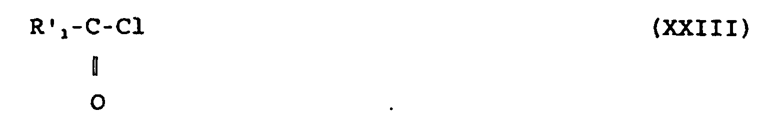 dans laquelle R'₁ a la signification indiquée ci-dessus, pour obtenir le produit de formule (XXIV) : 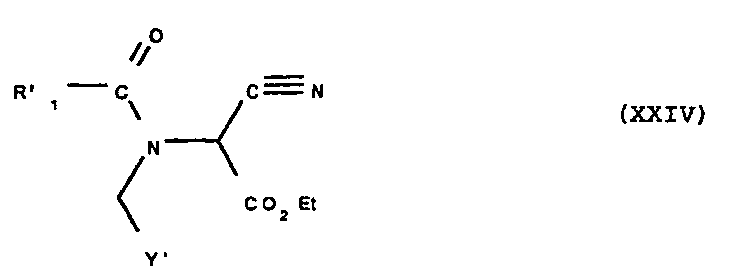 dans laquelle Y' et R'₁ ont les significations indiquées ci-dessus, que l'on soumet à un composé de formule (XXV) : 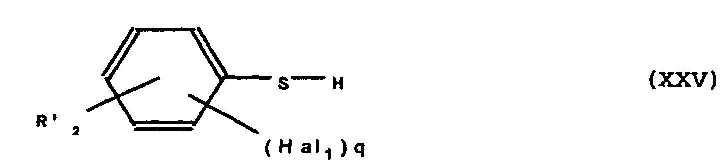 dans laquelle R'₂, Hal₁ et q ont la signification indiquée ci-dessus pour obtenir le produit de formule (XXVI) : 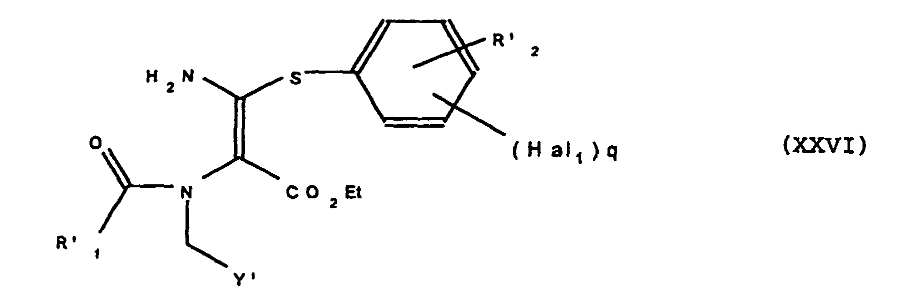 dans laquelle Y', R'₁, R'₂, Hal₁ et q ont les significations indiquées ci-dessus,
qui est cyclisé en produit de formule (I₂) : 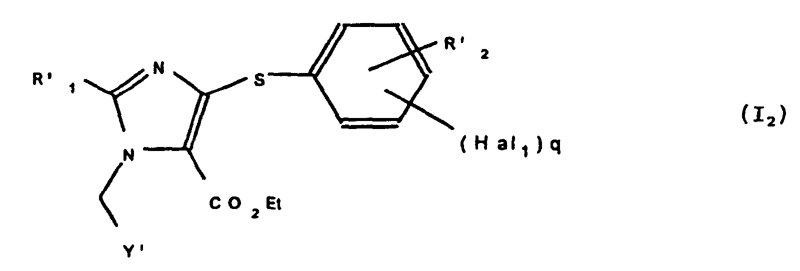 dans laquelle Y', R'₁, R'₂, Hal₁ et q ont les significations indiquées ci-dessus,
produits de formules (IX), (XI), (XIII), (XIV), (XV) et (I₂) qui peuvent être des produits de formule (I) et que, pour obtenir des ou d'autres produits de formule (I), l'on peut soumettre, si désiré et si nécessaire, à l'une ou plusieurs des réactions de transformations suivantes, dans un ordre quelconque :
a) une réaction d'estérification de fonction acide,
b) une réaction de saponification de fonction ester en fonction acide,
c) une réaction de transformation de fonction carboxy libre ou estérifié en radical formyle,
d) une réaction de transformation de la fonction cyano en fonction acide,
e) une réaction d'oxydation de groupement alkylthio en sulfoxyde ou sulfone correspondant,
f) une réaction de réduction de la fonction carboxy libre ou estérifié en fonction alcool,
g) une réaction de transformation de fonction alcoxy en fonction hydroxyle, ou encore de fonction hydroxyle en fonction alcoxy,
h) une réaction d'oxydation de fonction alcool en fonction aldéhyde, acide ou cétone,
i) une réaction de transformation du radical formyle en radical carbamoyle,
j) une réaction de transformation du radical carbamoyle en radical nitrile,
k) une réaction de transformation de radical nitrile en tétrazolyle,
l) une réaction de transformation d'une fonction halogénée en fonction formyle ou carboxy estérifié,
m) une réaction de transformation d'un radical formyle en fonction CH₂-CO₂alk ou CH=CH-CO₂alk dans laquelle alk représente un radical alkyle renfermant de 1 à 4 atomes de carbone, puis le cas échéant, transformation en acide correspondant,
n) une réaction de transformation d'un radical formyle en radical 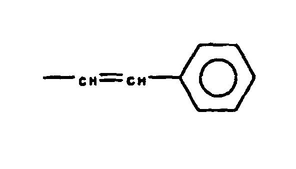 puis le cas échéant en 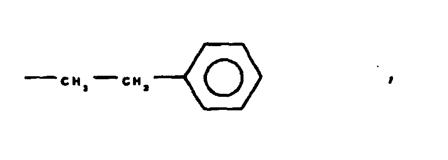
o) une réaction d'oxydation du radical S-alk en 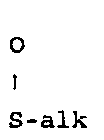 puis transformation en fonction SH et le cas échéant en S-A' dans lequel alk et A' ont la signification indiquée précédemment,
p) une réaction d'élimination des groupements protecteurs que peuvent porter les fonctions réactives protégées,
q) une réaction de salification par un acide minéral ou organique ou par une base pour obtenir le sel correspondant,
r) une réaction de dédoublement des formes racémiques en produits dédoublés,
lesdits produits de formule (I) ainsi obtenus étant sous toutes les formes isomères possibles racémiques, énantiomères et diastéréoisomères.

4. A titre de médicaments, les produits de formule (I) telle que définie aux revendications 1 et 2, ainsi que les sels d'addition avec les acides minéraux et organiques ou avec les bases minérales et organiques pharmaceutiquement acceptables desdits produits de formule (I).

5. Les compositions pharmaceutiques contenant à titre de principe actif, l'un au moins des médicaments tels que définis à la revendication 2.

6. Utilisation des produits de formule (I) telle que définie aux revendications 1 et 2, pour la préparation de compositions pharmaceutiques destinées au traitement d'affections résultant d'une stimulation anormale des récepteurs de l'endothéline et notamment destinées au traitement de l'hypertension induite par l'endothéline, de tous les spasmes vasculaires, au traitement des suites d'une hémorragie cérébrale, des insuffisances rénales, de l'infarctus du myocarde, de l'insuffisance cardiaque, à la prévention des resténoses post-angioplastie ainsi que des fibroses cardiaques et vasculaires.

## Patentansprüche

1. Produkte der Formel (I) 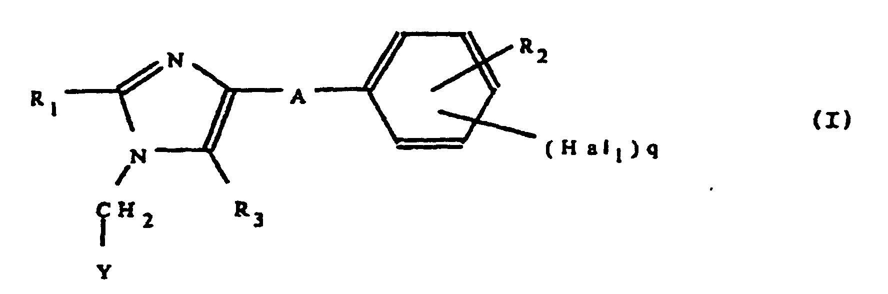 in der
R₁ einen Rest Ethyl, n-Propyl, Isopropyl, n-Butyl, Isobutyl oder tert.-Butyl darstellt,
A ein Schwefelatom ist,
R₂ einen linearen oder verzweigten Rest Alkyl oder Alkoxy mit höchstens 4 Kohlenstoffatomen darstellt, substituiert durch einen freien, in Salz überführten oder veresterten Rest Carboxy,
q gleich 0 ist,
R₃ einen freien, in Salz überführten oder veresterten Rest Carboxy oder einen freien oder in Salz überführten Rest Tetrazolyl darstellt,
Y einen Rest Phenyl darstellt, substituiert durch einen Rest Dioxol und gegebenenfalls durch ein Halogenatom,
wobei die genannten Produkte der Formel (I) in allen ihren möglichen isomeren racemischen Formen, Enantiomeren und Diastereoisomeren vorliegen, sowie die Additionssalze der genannten Produkte der Formel (I) mit Mineralsäuren und organischen Säuren oder mit Mineralbasen und organischen Basen.

2. Die folgenden Produkte:
- 4-{[4-(Carboxymethoxy)-phenyl]-thio}-1-[(6-chlor-1,3-benzodioxol-5-yl)-methyl]-2-propyl-1H-imidazol-5-carbonsäure,
- 4-{[4-(2-Carboxyethyl)-phenyl]-thio}-1-[(6-chlor-1,3-benzodioxol-5-yl)-methyl]-2-(1-methylethyl)-1H-imidazol-5-carbonsäure,
- 4-[(4-Carboxyphenyl)-thio]-1-[(6-chlor-1,3-benzodioxol-5-yl)-methyl]-2-propyl-1H-imidazol-5-carbonsäure.

3. Verfahren zur Herstellung der Produkte der Formel (I) wie in Anspruch 1 definiert, **dadurch gekennzeichnet, daß** man
entweder eine Verbindung der Formel (II) 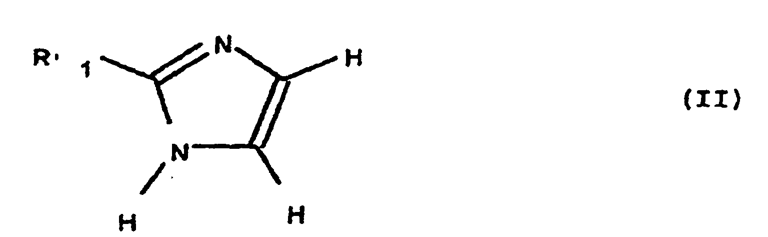 in der R'₁ die in Anspruch 1 für R₁ angegebene Bedeutung besitzt, in der die eventuellen reaktiven Funktionen gegebenenfalls durch Schutzgruppen geschützt sind,
einer Reaktion mit einer Verbindung der Formel (III) 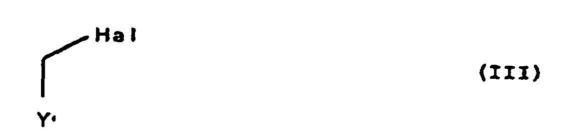 unterzieht, in der Hal ein Halogenatom ist und Y' die in Anspruch 1 für Y angegebene Bedeutung besitzt, in der die eventuellen reaktiven Funktionen gegebenenfalls durch Schutzgruppen geschützt sind, um ein Produkt der Formel (IV) 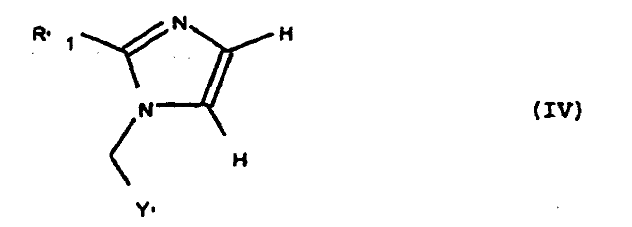 zu erhalten, in der R'₁ und Y' die oben angegebenen Bedeutungen besitzen, und man das Produkt der Formel (IV) einer Reaktion zur Halogenierung unter ziehen kann, um das Produkt der Formel (V) 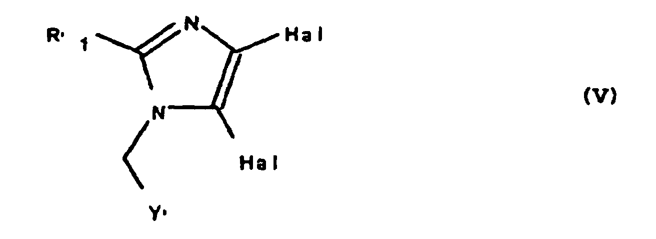 zu erhalten, in der R'₁, Hal und Y' die oben angegebenen Bedeutungen besitzen,
oder eine Verbindung der Formel (VI) 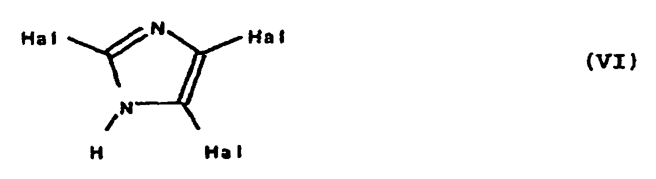 in der Hal die oben angegebene Bedeutung besitzt,
entweder einer Reaktion mit der wie oben definierten Verbindung der Formel (III) oder der Einwirkung einer Schutzgruppe P unterzieht, um ein Produkt der Formel (VII) 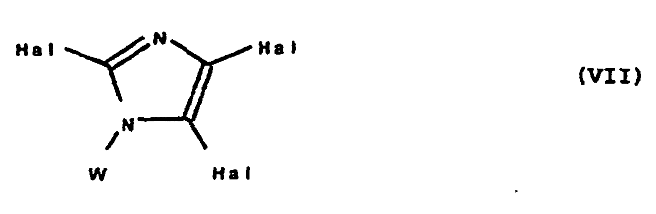 zu erhalten,
in der Hal die oben angegebene Bedeutung besitzt und W entweder -CH₂-Y' darstellt mit Y' wie oben definiert oder P bedeutet, das eine Schutzgruppe für das Stickstoffatom ist,
und man das Produkt der Formel (VII) einer Reaktion zum Austausch Halogen-Metall und anschließend einer Reaktion mit Dimethylformamid oder einer elektrophilen Verbindung der Formel (VIIIₐ) oder (VIII_{b})
L₁-CHO (VIIIₐ)
L₁-COCl (VIII_{b})
unterzieht, worin L₁ einen linearen oder verzweigten Rest Alkyl mit höchstens 6 Kohlenstoffatomen darstellt, der gegebenenfalls substituiert ist durch einen Rest Alkoxy oder geschützten Rest Hydroxyl, um ein Produkt der Formel (IX) 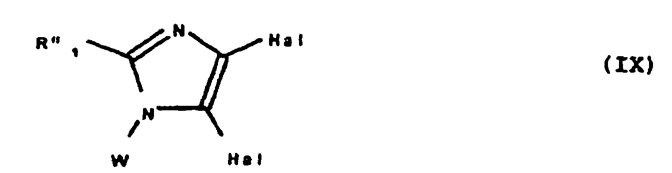 zu erhalten, in der Hal und W die oben angegebenen Bedeutungen besitzen, R"₁ einen Rest Alkylcarbonyl, Formyl oder Hydroxyalkyl darstellt, worin der Rest Alkyl die oben angegebene Bedeutung besitzt und worin die eventuellen reaktiven Funktionen gegebenenfalls durch Schutzgruppen geschützt sind,
und man die Produkte der Formeln (V) und (IX) einer Reaktion zum Austausch Halogen-Metall an einem der Halogenatome und anschließend einer Reaktion mit CO₂ oder Dimethylformamid oder einer elektrophilen Verbindung der Formel (X) 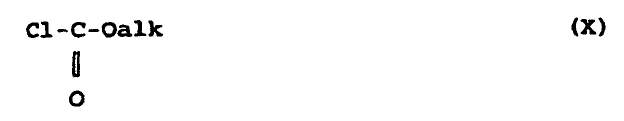 unterziehen kann, in der alk einen Rest Alkyl mit höchstens 4 Kohlenstoffatomen darstellt,
um die Verbindung der Formel (XI) 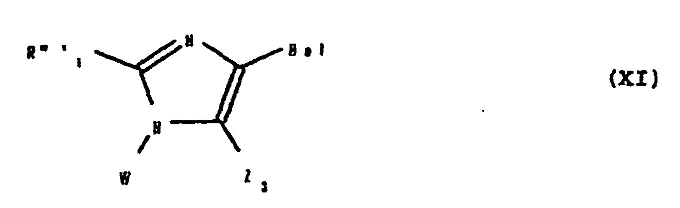 zu erhalten, in der R'' '_{1,} U, R'₁ oder R"₁ darstellt wie oben definiert, Hal, Y' und W die oben angegebenen Bedeutungen besitzen und Z den freien oder veresterten Rest Carboxy oder den Rest Formyl darstellt,
und man die Verbindung der Formel (XI) einer Reaktion mit einer Verbindung der Formel (XII) 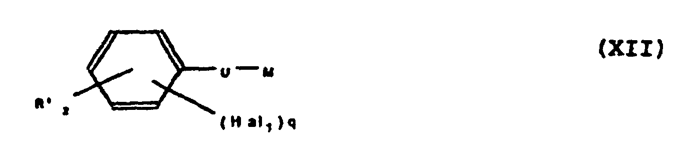 unterziehen kann, in der U ein Atom von Schwefel oder Sauerstoff darstellt, M ein Metall wie Natrium, Kalium oder Kupfer ist, Hal₁ und q die bei Anspruch 1 angegebene Bedeutung besitzen, R'₂ R₂ darstellt wie in Anspruch 1 definiert, worin die eventuellen reaktiven Funktionen gegebenenfalls durch Schutzgruppen geschützt sind, um die Verbindung der Formel (XIII) 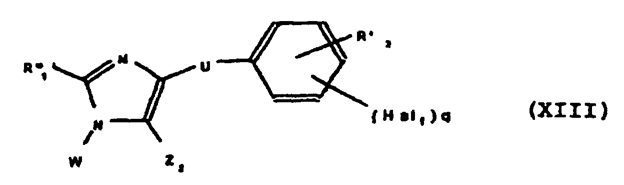 zu erhalten, in der R'' '₁, U, R'₂, Hal₁, q und W die oben angegebenen Bedeutungen besitzen, und man das Produkt der Formel (XIII), wenn Z₃ den Rest Formyl darstellt, einer Reaktion zur Oxidation unterziehen kann, um das Produkt der Formel (XIV) 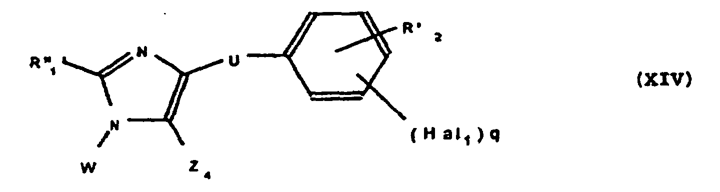 zu erhalten, in der R'' '₁, U, R'₂, Hal₁, q und W die oben angegebenen Bedeutungen besitzen und Z₄ den Rest Cyano oder den freien oder durch einen linearen oder verzweigten Rest Alkyl mit höchstens 6 Kohlenstoffatomen veresterten Rest Carboxy darstellt, und man die Produkte der Formeln (XIII) oder (XIV) in dem Fall, wo W P darstellt wie oben definiert, und nach Freisetzung der durch P, wie oben definiert, blockierten Aminfunktion mit der wie oben definierten Verbindung der Formel (III) zur Reaktion bringen kann, um ein Produkt der Formel (XV) 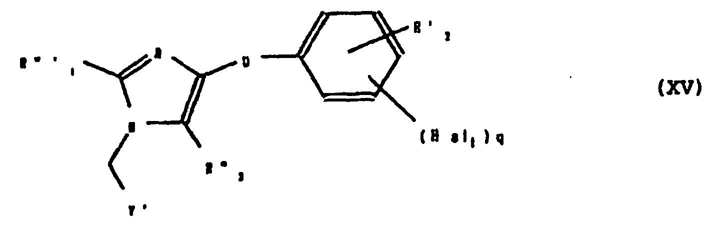 zu erhalten, in der R'' '₁, U, R'₂, Hal₁, q und Y' die oben angegebenen Bedeutungen besitzen und R"₃ Z₃ oder Z₄ darstellt wie oben definiert,
oder eine Verbindung der Formel (XX)
Y'-CHO (XX)
in der Y' die oben angegebene Bedeutung besitzt, der Einwirkung einer Verbindung der Formel (XXI) 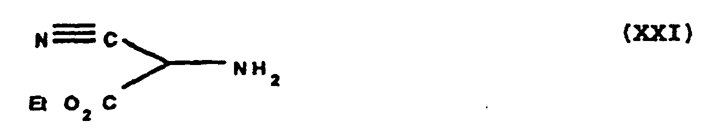 unterzieht,
um ein Produkt der Formel (XXII) 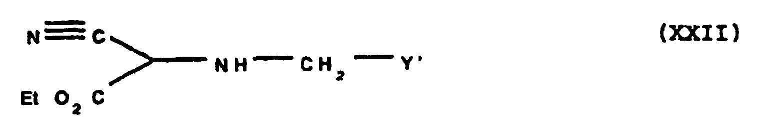 zu erhalten, in der Y' die oben angegebene Bedeutung besitzt, die man der Einwirkung einer Verbindung der Formel (XXIII) 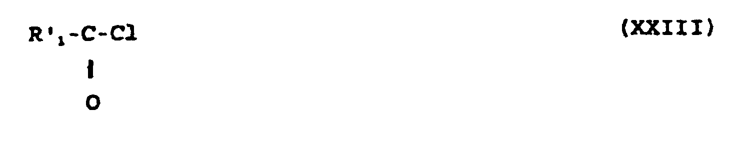 unterzieht, in der R'₁ die oben angegebene Bedeutung besitzt, um das Produkt der Formel (XXIV) 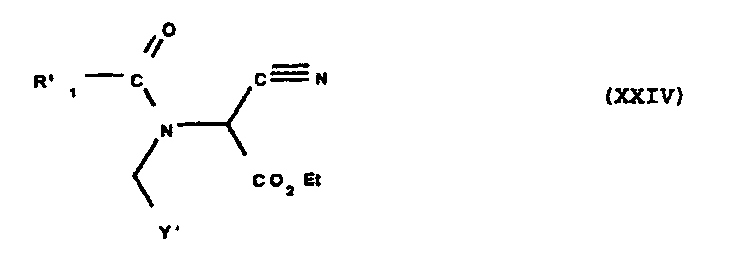 zu erhalten, in der Y' und R'₁ die oben angegebenen Bedeutungen besitzen, die man der Einwirkung einer Verbindung der Formel (XXV) 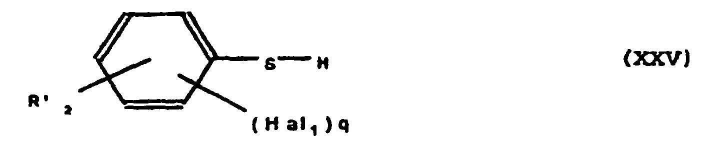 unterzieht, in der R'₂, Hal₁ und q die oben angegebenen Bedeutungen besitzen, um das Produkt der Formel (XXVI) 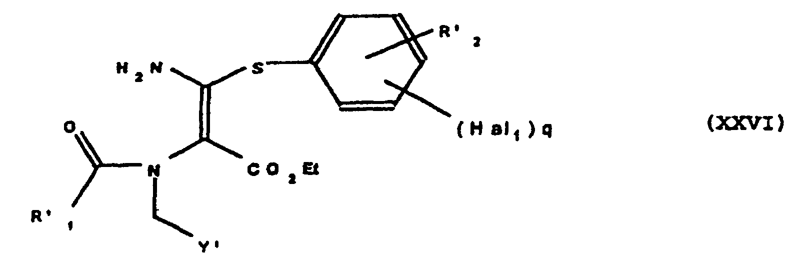 zu erhalten, in der Y', R'₁, R'₂, Hal₁ und q die oben angegebenen Bedeutungen besitzen, das zu einem Produkt der Formel (I₂) 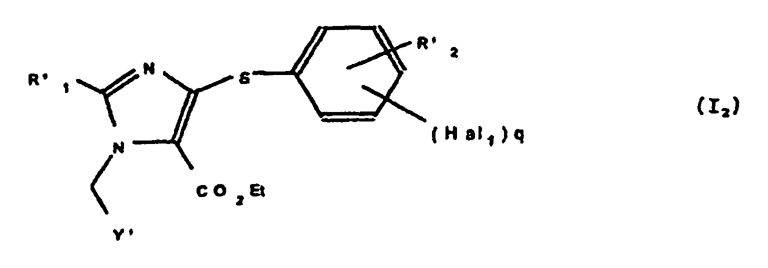 cyclisiert wird, in der Y', R'₁, R'₂, Hal₁ und q die oben angegebenen Bedeutungen besitzen,
und man die Produkte der Formeln (IX), (XI), (XIII), (XIV), (XV) und (I₂), die Produkte der Formel (I) sein können, um diese oder andere Produkte der Formel (I) zu erhalten, wenn erwünscht und wenn erforderlich, einer oder mehreren der folgenden Umwandlungsreaktionen in irgendeiner Reihenfolge unterziehen kann:
a) einer Reaktion zur Veresterung der Säurefunktion,
b) einer Reaktion zur Verseifung der Esterfunktion zur Säurefunktion,
c) einer Reaktion zur Umwandlung der freien oder veresterten Carboxyfunktion zum Rest Formyl,
d) einer Reaktion zur Umwandlung der Cyanofunktion zur Säurefunktion,
e) einer Reaktion zur Oxidation der Gruppe Alkylthio zum entsprechenden Sulfoxid oder Sulfon,
f) einer Reaktion zur Reduktion der freien oder veresterten Carboxyfunktion zur Alkoholfunktion,
g) einer Reaktion zur Umwandlung der Alkoxyfunktion zur Hydroxylfunktion oder auch der Hydroxylfunktion zur Alkoxyfunktion,
h) einer Reaktion zur Oxidation der Alkoholfunktion zur Aldehydfunktion, Säurefunktion oder Ketonfunktion,
i) einer Reaktion zur Umwandlung des Restes Formyl zum Rest Carbamoyl,
j) einer Reaktion zur Umwandlung des Restes Carbamoyl zum Rest Nitril,
k) einer Reaktion zur Umwandlung des Restes Nitril zum Rest Tetrazolyl,
l) einer Reaktion zur Umwandlung einer halogenierten Funktion zu einer Formylfunktion oder veresterten Carboxyfunktion,
m) einer Reaktion zur Umwandlung eines Restes Formyl zur Funktion CH₂-CO₂alk oder CH=CH-CO₂alk, worin alk einen Rest Alkyl mit 1 bis 4 Kohlenstoffatomen darstellt, und anschließend gegebenenfalls Umwandlung zur entsprechenden Säure,
n) einer Reaktion zur Umwandlung eines Restes Formyl zum Rest 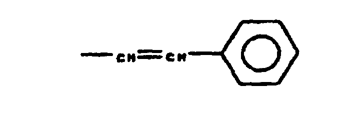 anschließend gegebenenfalls zum Rest 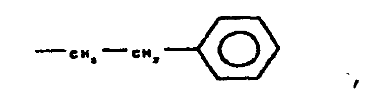
o) einer Reaktion zur Oxidation des Restes S-alk zum Rest 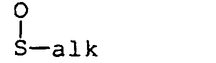 und anschließend Umwandlung zur Funktion SH und gegebenenfalls zur Funktion S-A', worin alk und A' die oben angegebenen Bedeutungen besitzen,
p) einer Reaktion zur Entfernung der Schutzgruppen, die von den geschützten reaktiven Funktionen getragen werden können,
q) einer Reaktion zur Salzbildung durch eine Mineralsäure oder organische Säure oder durch eine Base, um das entsprechende Salz zu erhalten,
r) einer Reaktion zur Aufspaltung der racemischen Formen in die Spaltprodukte,
wobei die auf diese Weise erhaltenen Produkte der Formel (I) in allen ihren möglichen isomeren racemischen Formen, Enantiomeren und Diastereoisomeren vorliegen.

4. Als Arzneimittel die Produkte der Formel (I) wie in den Ansprüchen 1 und 2 definiert sowie die Additionssalze der genannten Produkte der Formel (I) mit pharmazeutisch akzeptablen Mineralsäuren und organischen Säuren oder mit Mineralbasen und organischen Basen.

5. Pharmazeutische Zusammensetzungen, enthaltend als Wirkstoff mindestens eines der wie in Anspruch 2 definierten Arzneimittel.

6. Verwendung der Produkte der Formel (I) wie in den Ansprüchen 1 und 2 definiert, zur Herstellung von pharmazeutischen Zusammensetzungen, vorgesehen für die Behandlung von Erkrankungen, die aus einer anormalen Stimulierung der Rezeptoren des Endothelins resultieren, und insbesondere vorgesehen für die Behandlung von durch Endothelin induziertem Bluthochdruck, von allen vasculären Spasmen, für die Behandlung der Folgen einer Gehirnblutung, von Niereninsuffizienzen, von Myokardinfarkt, von Herzinsuffizienz, zur Vorbeugung von post-angioplastischen Restenosen sowie von Herz- und Gefäßfibrosen.

## Claims

1. Products of formula (I): 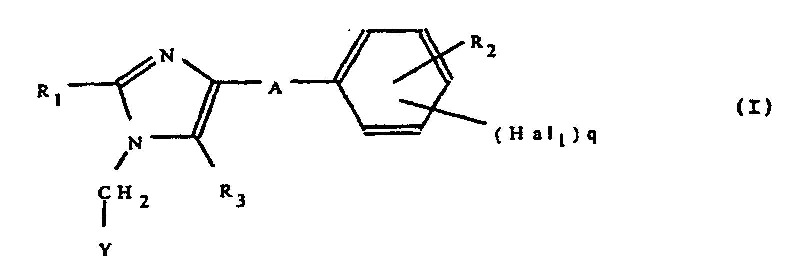 in which:
R₁ represents an ethyl, n-propyl, isopropyl, n-butyl, isobutyl or tert-butyl radical,
A represents a sulphur atom,
R₂ represents a linear or branched alkyl or alkoxy radical which includes at most 4 carbon atoms and which is substituted by a free, salified or esterified carboxyl radical,
q is equal to 0,
R₃ represents a free, salified or esterified carboxyl radical or a free or salified tetrazolyl radical,
Y represents a phenyl radical substituted by a dioxolyl radical and optionally by a halogen atom, the said products of formula (I) being in all the isomeric forms possible, racemic, enantiomeric and diastereoisomeric, and the addition salts with inorganic and organic acids or with inorganic and organic bases of the said products of formula (I).

2. The following products:
- 4-((4-(carboxymethyl)phenyl)thio)-1-((6-chloro-1,3-benzodioxol-5-yl)methyl)-2-propyl-1H-imidazole-5-carboxylic acid,
- 4-((4-(2-carboxyethyl)phenyl)thio)-1-((6-chloro-1,3-benzodioxol-5-yl)methyl)-2-(1-methylethyl)-1H-imidazole-5-carboxylic acid,
- 4-((4-carboxyphenyl)thio)-1-((6-chloro-1,3-benzodioxol-5-yl)methyl)-2-propyl-1H-imidazole-5-carboxylic acid.

3. Process for the preparation of the products of formula (I) as defined in Claim 1, **characterized in that**:
either a compound of formula (II): 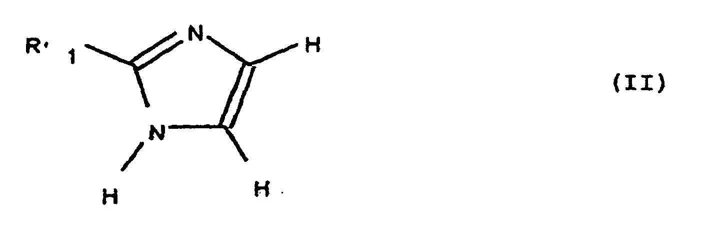 in which R'₁ has the meaning indicated in Claim 1 for R₁, in which the possible reactive functional groups are optionally protected by protective groups,
is reacted with the compound of formula (III): 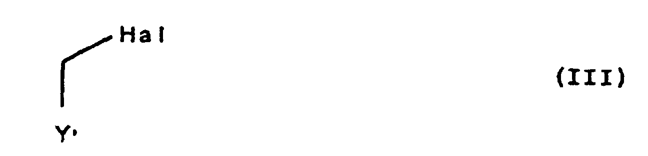 in which Hal represents a halogen atom and Y' has the meaning indicated in Claim 1 for Y, in which the possible reactive functional groups are optionally protected by protective groups, to produce the product of formula (IV): 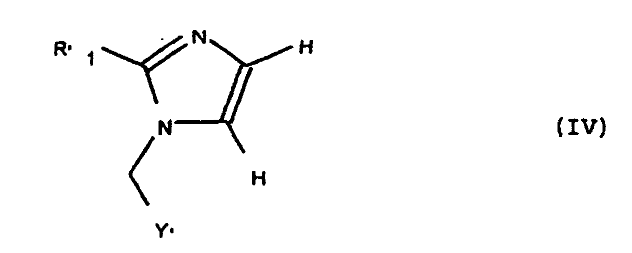 in which R'₁ and Y' have the meanings indicated above, which product of formula (IV) can be subjected to a halogenation reaction to produce the product of formula (V): 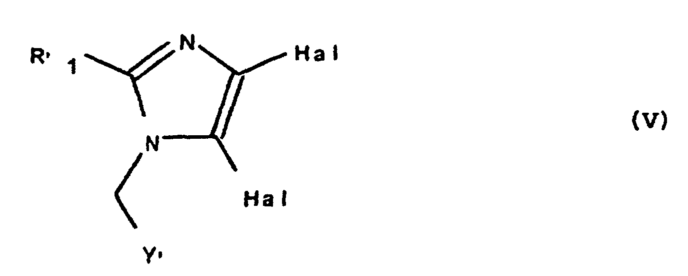 in which R'₁, Hal and Y' have the meanings indicated above,
or a compound of formula (VI): 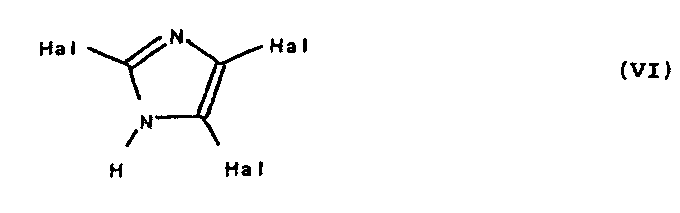 in which Hal has the meaning indicated above, is either reacted with the compound of formula (III) as defined above or is subjected to the action of a protective group P, to produce a product of formula (VII): 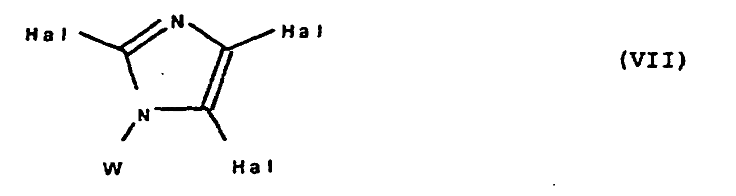 in which Hal has the meaning indicated above and W represents either -CH₂-Y', with Y' as defined above, or P, which represents a protective group for the nitrogen atom, which product of formula (VII) is subjected to a halogen-metal exchange reaction and is then reacted with dimethylformamide or with an electrophile of formula (VIIIₐ) or (VIII_{b}):
L₁-CHO (VIIIₐ)
L₁-CO-Cl (VIII_{b})
in which L₁ represents a linear or branched alkyl radical which includes at most 6 carbon atoms and which is optionally substituted by an alkoxy or protected hydroxyl radical, to produce a product of formula (IX): 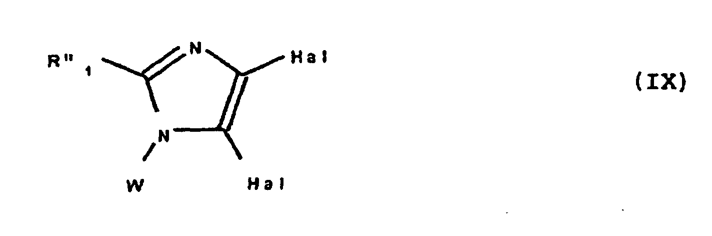 in which Hal and W have the meanings indicated above and R"₁ represents an alkylcarbonyl, formyl or hydroxyalkyl radical in which the alkyl radical has the meaning indicated above and in which the possible reactive functional groups are optionally protected by protective groups, which products of formulae (V) and (IX) can be subjected to a halogen-metal exchange reaction with one of the halogen atoms and can then be reacted with CO₂ or dimethylformamide or an electrophilic compound of formula (X): 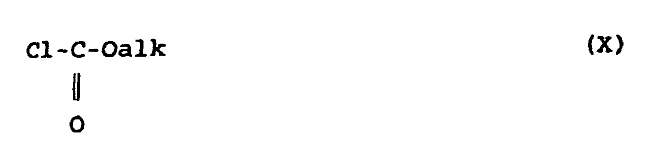 in which alk represents an alkyl radical including at most 4 carbon atoms,
to produce the compound of formula (XI): 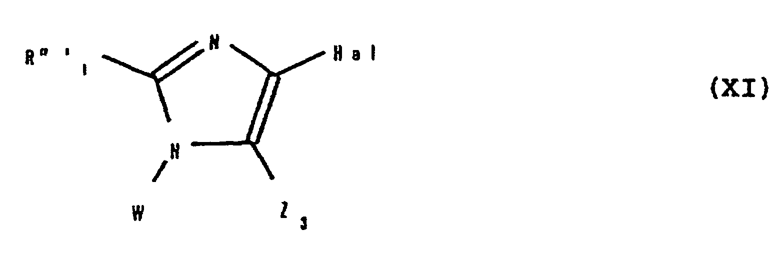 in which R'''₁ represents R'₁ or R"₁ as defined above, Hal, Y' and W have the meanings indicated above and Z₃ represents the free or esterified carboxyl radical or the formyl radical,
which compound of formula (XI) can be reacted with a compound of formula (XII): 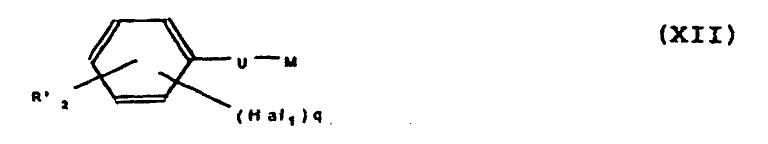 in which U represents a sulphur atom, M represents a metal, such as sodium, potassium or copper, Hal₁ and q have the meanings indicated in Claim 1 and R'₂ represents R₂ as defined in Claim 1, in which the possible reactive functional groups are optionally protected by protective groups, to produce the compound of formula (XIII): 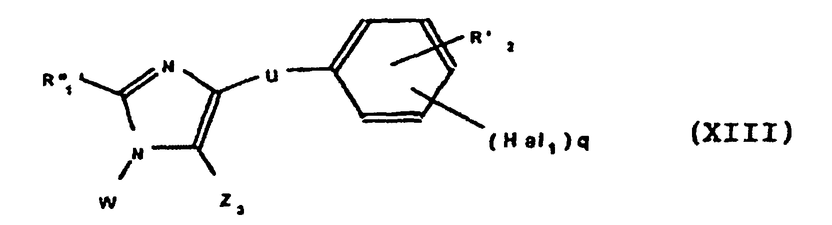 in which R"'₁, U, R'₂, Hal₁, q and W have the meanings indicated above, which product of formula (XIII), when Z₃ represents the formyl radical, can be subjected to an oxidation reaction, to produce the product of formula (XIV): 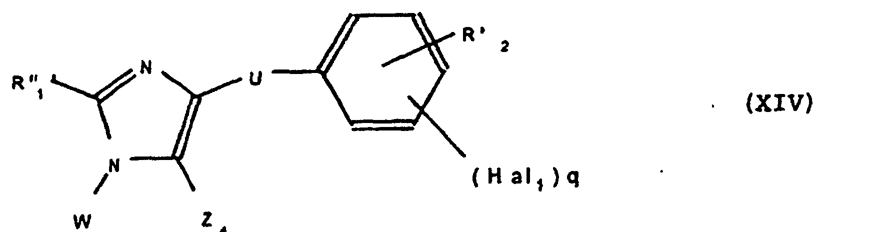 in which R'''₁, U, R'₂, Hal₁, q and W have the meanings indicated above and Z₄ represents the cyano radical, the free carboxyl radical or the carboxyl radical esterified by a linear or branched alkyl radical including at most 6 carbon atoms, which products of formula (XIII) or (XIV), in the case where W represents P as defined above and after release of the amine functional group blocked by P as defined above, are reacted with the compound of formula (III) as defined above, to produce a product of formula (XV): 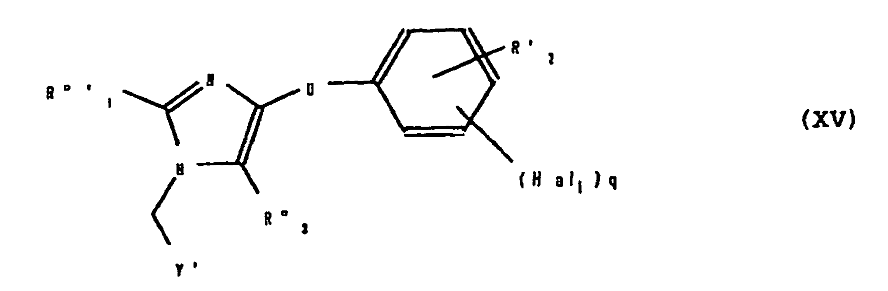 in which R'' '₁, U, R'₂, Hal₁, q and Y' have the meanings indicated above and R"₃ represents Z₃ or Z₄ as defined above,
or a compound of formula (XX):
Y'-CHO (XX)
in which Y' has the meaning indicated above, is subjected to a compound of formula (XXI): 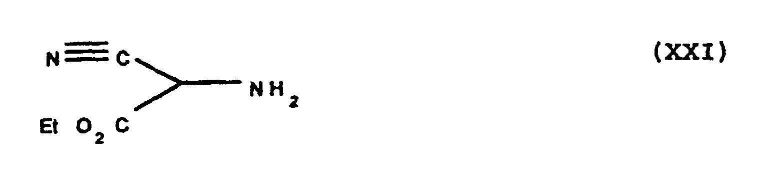 to produce a product of formula (XXII): 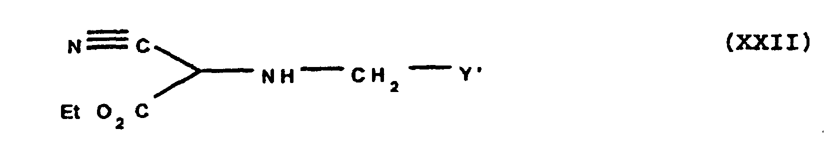 in which Y' has the meaning indicated above, which is subjected to a compound of formula (XXIII): 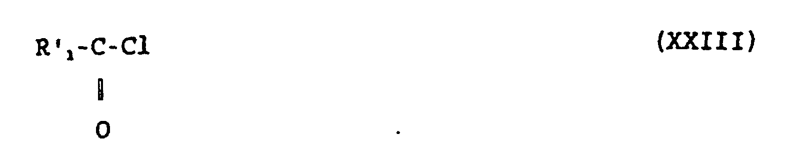 in which R'₁ has the meaning indicated above, to produce the product of formula (XXIV): 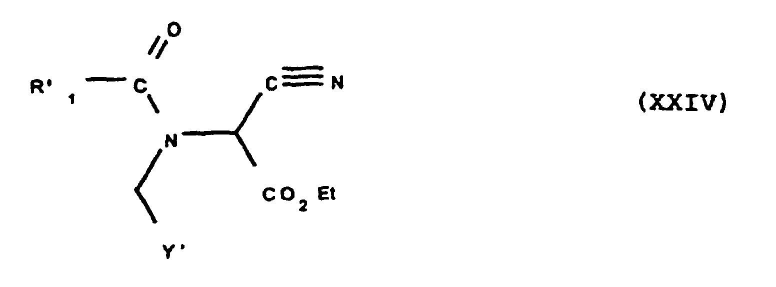 in which Y' and R'₁ have the meanings indicated above, which is subjected to a compound of formula (XXV): 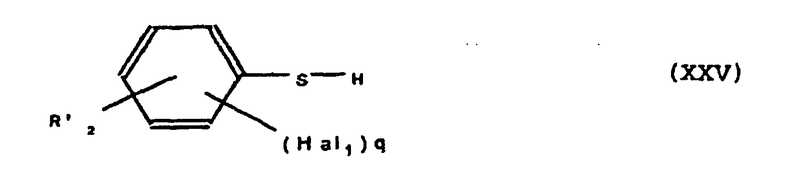 in which R'₂, Hal₁ and q have the meanings indicated above, to produce the product of formula (XXVI) : 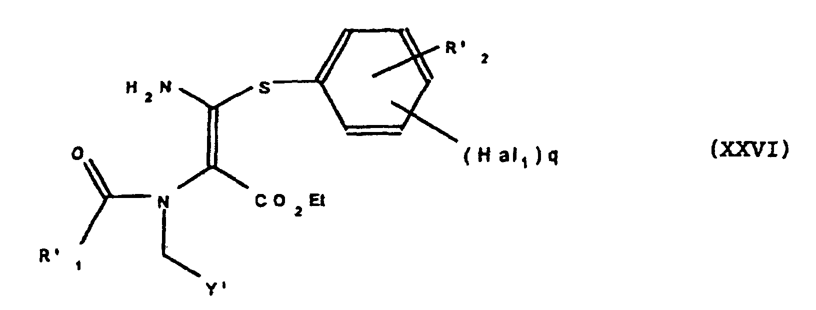 in which Y', R'₁, R'₂, Hal₁ and q have the meanings indicated above,
which is cyclized to give the product of formula (I₂) : 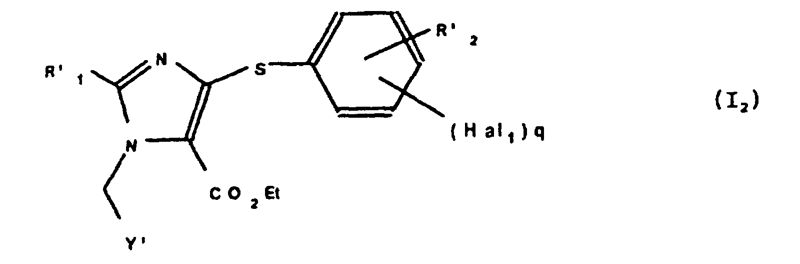 in which Y', R'₁, R'₂, Hal₁ and q have the meanings indicated above,
which products of formulae (IX), (XI), (XIII), (XIV), (XV) and (I₂) can be products of formula (I) and which, to produce products or other products of formula (I), can be subjected, if desired and if necessary, to one or more of the following conversion reactions, in any order:
a) a reaction for esterification of an acid functional group,
b) a reaction for saponification of an ester functional group to an acid functional group,
c) a reaction for conversion of a free or esterified carboxyl functional group to a formyl radical,
d) a reaction for conversion of the cyano functional group to an acid functional group,
e) a reaction for oxidation of an alkylthio group to the corresponding sulphoxide or sulphone,
f) a reaction for reduction of the free or esterified carboxyl functional group to an alcohol functional group,
g) a reaction for conversion of an alkoxy functional group to a hydroxyl functional group or alternatively of a hydroxyl functional group to an alkoxy functional group,
h) a reaction for oxidation of an alcohol functional group to an aldehyde, acid or ketone functional group,
i) a reaction for conversion of the formyl radical to a carbamoyl radical,
j) a reaction for conversion of the carbamoyl radical to a nitrile radical,
k) a reaction for conversion of a nitrile radical to tetrazolyl,
l) a reaction for conversion of a halogenated functional group to a formyl or esterified carboxyl functional group,
m) a reaction for conversion of a formyl radical to a CH₂-CO₂alk or CH=CH-CO₂alk functional group, in which alk represents an alkyl radical including from 1 to 4 carbon atoms, and then, if appropriate, conversion to the corresponding acid,
n) a reaction for conversion of a formyl radical to a 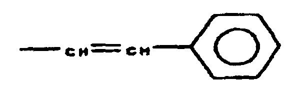 radical and then, if appropriate, to 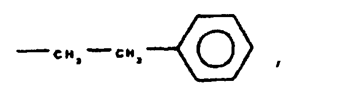
o) a reaction for oxidation of the S-alk radical to 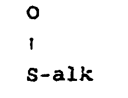 and then conversion to an SH functional group and, if appropriate, to S-A', in which alk and A' have the meanings indicated above,
p) a reaction for removal of the protective groups which the protected reactive functional groups can carry,
q) a reaction for salification by an inorganic or organic acid or by a base, to produce the corresponding salt,
r) a reaction for resolution of the racemic forms to give resolved products,
the said products of formula (I) thus obtained being in all the isomeric forms possible, racemic, enantiomeric and diastereoisomeric.

4. As medicaments, the products of formula (I) as defined in Claims 1 and 2, and the addition salts with pharmaceutically acceptable inorganic and organic acids or with pharmaceutically acceptable inorganic and organic bases of the said products of formula (I).

5. The pharmaceutical compositions comprising, as active principle, at least one of the medicaments as defined in Claim 2.

6. Use of the products of formula (I) as defined in Claims 1 and 2 in the preparation of pharmaceutical compositions intended for the treatment of conditions resulting from abnormal stimulation of endothelin receptors and intended in particular for the treatment of endothelin-induced hypertension, of all vascular spasms, of the effects of a cerebral haemorrhage, of renal insufficiency, of myocardial infarction or of cardiac insufficiency, in the prevention of post-angioplastic restenosis, and cardiac and vascular fibrosis.
